# EUROPEAN PATENT SPECIFICATION

(11) **EP 4 519 245 B1**
(45) Date of publication and mention of the grant of the patent: **11.11.2026**
(21) Application number: 23722920.8
(22) Date of filing: 03.05.2023
(51) Int. Cl.: C07D 205/04, A61P 25/00, A61K 31/397

(54) **AZETIDINE COMPOUNDS, PHARMACEUTICAL COMPOSITIONS THEREOF, PREPARATION METHODS AND THEIR USES IN THE TREATMENT OF CNS DISORDERS**
AZETIDINVERBINDUNGEN, PHARMAZEUTISCHE ZUSAMMENSETZUNGEN DARAUS, HERSTELLUNGSVERFAHREN UND IHRE VERWENDUNGEN BEI DER BEHANDLUNG VON ZNS-ERKRANKUNGEN
COMPOSÉS D'AZÉTIDINE, COMPOSITIONS PHARMACEUTIQUES DE CEUX-CI, PROCÉDÉS DE PRÉPARATION ET LEURS UTILISATIONS DANS LE TRAITEMENT DE TROUBLES DU SNC

(30) Priority: 05.05.2022 FI 20225388
(43) Date of publication of application: 12.03.2025
(73) Proprietor: Equinorm Ltd, 02150 Espoo (FI)
(72) Inventor: TAKIO, Ville Emil, 02150 Espoo (FI); HEIKKINEN, Jenni, Dr, 02150 Espoo (FI)
(74) Representative: Laine IP Oy
(86) International application number: PCT/FI2023/050241
(87) International publication number: WO 2023/214119

(56) References cited:
- WO-A1-2006/134487
- WO-A1-2010/058020
- WO-A1-2019/234115
- GB-A- 1 236 078
- HE ZHI-TAO ET AL: "Palladium-catalyzed [alpha]-arylation for the addition of small rings to aromatic compounds", NATURE COMMUNICATIONS, vol. 10, no. 1, 9 September 2019 (2019-09-09), XP093056488, Retrieved from the Internet <URL:https://www.nature.com/articles/s41467-019-12090-z.pdf> DOI: 10.1038/s41467-019-12090-z
- DATABASE REGISTRY [online] CHEMICAL ABSTRACT SERVICE, COLUMBUS, OHIO, US; 29 December 2021 (2021-12-29), COMPOUNDS FROM CHEMCATS CATALOG: "azetidine", XP093056691, Database accession no. 2751306-39-3

## Description

### TECHNICAL FIELD

The present invention relates to novel compounds derived from 3,3-disubstituted azetidines and pharmaceutical compositions comprising said compounds. The invention also relates to said compounds for use in the treatment or prevention of drug addiction or a CNS related disease or condition, in addition to methods for the preparation of said compounds.

### BACKGROUND

Azacyclobutane (azetidine) is a heterocyclic compound containing three carbon atoms and one nitrogen atom. Azetidine and its derivatives are relatively rare structural motifs in natural products. They are a component of mugineic acids and penaresidins. An azetidine containing natural product is azetidine-2-carboxylic acid, which is a toxic mimic of proline.

EA202092955A1 discloses certain 3,3-disubstituted azetidines as LPAl receptor modulators in the treatment of fibrosis.

US2012232026A1 discloses as antiparasitic agents certain 3,3-disubstituted azetidines that are spirocyclic isoxazoline derivatives.

WO2005049605A1 discloses antibacterial aminoquinazolidinedione derivatives, wherein some of the derivatives contain a 3,3-disubstituted azetidine-1-yl group.

WO2016193241A1 discloses certain 3,3-disubstituted azetidines having muscarinic receptor antagonist and beta2 adrenergic receptor agonist activity.

WO2016177849A1 discloses certain compounds containing a 3,3-disubstituted azetidine-1-yl group, wherein the compounds are both phosphodiesterase 4 (PDE4) enzyme inhibitors and muscarinic M3 receptor antagonists for use in the prevention and/or treatment asthma or COPD. WO 2010/058020 A1 discloses certain 1 - alkyl- 3 -hydroxy- 3 -phenylazetidine derivatives useful as modulators of cortical catecholaminergic neurotransmission. GB 1236078 A discloses certain substituted azetidinols that have pharmacological activity especially on the central nervous system as antidepressants.

TAAR1 (trace amine associated receptor 1) is a protein that in humans is encoded by the *TAAR1* gene. TAAR1 plays a role in regulating neurotransmission in dopamine, norepinephrine, and serotonin neurons in the CNS and it affects immune system and neuroimmune system function through different mechanisms. Among endogenous ligands of the human TAAR1 (hTAAR1) receptor are tyramine, β-phenethylamine (PEA), dopamine, and octopamine. TAAR1 is a high-affinity receptor for amphetamine, methamphetamine, and trace amines which mediates some of their cellular effects in monoamine neurons within the central nervous system. Low PEA concentration in the brain is associated with major depressive disorder, and high concentrations are associated with schizophrenia. In addition, low PEA levels and under-activation of TAAR1 seems to be associated with attention deficit hyperactivity disorder (ADHD). TAAR1-selective ligands have been proposed to have therapeutic potential for treating psychostimulant addictions (e.g., cocaine, amphetamine, methamphetamine).

The dopamine transporter (DAT) is a membrane-spanning protein that pumps the neurotransmitter dopamine out of the synaptic cleft back into cytosol. Therefore, DAT is implicated in a number of dopamine-related disorders, including ADHD, bipolar disorder, alcoholism, and substance use disorder (drug addiction, alcoholism). In addition, increased activity of DAT is linked to clinical depression.

Also dopamine receptors are involved in many neurological disorders, diseases, and conditions, including ADHD, Parkinson's disease (PD), schizophrenia, neuroleptic malignant syndrome, drug addiction, alcoholism, social phobia, and Tourette's syndrome. Dopamine receptors bind dopamine; while psychostimulants are typically dopamine receptors' indirect agonists, dopamine receptors antagonists often work as antipsychotics.

As dopamine receptors, also adrenergic receptors are a class of G protein-coupled receptors (GPCRs). Adrenergic receptors are targets of catecholamines like norepinephrine and epinephrine. FDA-approved drugs that bind to adrenergic receptors are used to treat e.g. hypertension, and ADHD, and agonists to β2-adrenergic receptors have been found to be effective in the treatment of PD.

Serotonin receptors are also GPCRs and are ligand-gated ion channels that are activated by the endogenous neurotransmitter serotonin. Serotonin receptors are found in the central and peripheral nervous systems. Various biological and neurological processes that serotonin receptors influence are e.g. aggression, anxiety, appetite, cognition, learning, memory, mood, nausea, sleep, and thermoregulation. Therefore, serotonin receptors are the target of drugs, such as antidepressants, antipsychotics, anorectics, antiemetics, gastroprokinetic agents, antimigraine agents, hallucinogens, and entactogens.

The NMDA receptor (N-methyl-D-aspartate receptor) is a glutamate receptor and ion channel found in neurons. The NMDA receptor may be important for controlling synaptic plasticity and mediating learning and memory functions. NMDA receptor inhibitors and antagonists are used for the treatment of Alzheimer's disease and depression, and antagonists are used as anasthetics.

Cholinergic receptors are receptors and integral membrane proteins that are activated by the neurotransmitter acetylcholine. Among cholinergic receptors are the nicotinic acetylcholine (nAChR) and muscarinic acetylcholine (mAChR) receptors.

Monoamine oxidase A (MAOA) is an enzyme that in humans is encoded by the *MAOA* gene. Diseases, disorders, and conditions associated with MAOA include Alzheimer's disease (AD), aggression, panic disorder, bipolar affective disorder, depression, ADHD, and antisocial behaviour.

Catechol-O-methyltransferase (COMT) is one of several enzymes that inactivates catecholamines such as the neurotransmitters dopamine, epinephrine, and norepinephrine, and catecholestrogens, all having a catechol structure, by introducing a methyl group to the catechol structure. COMT inhibitors are used for the treatment of PD.

Dopamine beta-hydroxylase (DBH) is an enzyme that catalyzes the conversion of dopamine to norepinephrine. DBH has been suggested as the target of drugs in diseases, conditions, and disorders such as drug addiction, alcoholism, ADHD, schizophrenia, and AD.

The µ-opioid receptors (MORs) are inhibitory GPCPs that have a high affinity for the endogenous peptides enkephalins and β-endorphin. MOR agonists are used for treating or preventing pain.

### SUMMARY

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter. The subject-matter of the present invention is disclosed in the appended claims.

An object of the present invention is to provide compounds useful in treating or preventing drug addiction or a CNS related disease or condition.

It has now surprisingly been found that compounds derived from 3,3-disubstituted azetidines show binding affinities to proteins associated to drug addiction or a CNS related disease or condition.

Disclosed are compounds of formula (I) 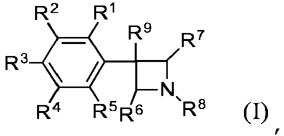 wherein
R¹, R², R³, R⁴, and R⁵ are each independently selected from the group consisting of H, halogen, C₁₋₄-alkyl, C₁₋₃- (per) haloalkyl, C₁₋₃-alkoxy, C₁₋₃- (per) haloalkoxy, and OH; or
R¹, R², and R⁵ are each independently selected from the group consisting of H, halogen, C₁₋₄-alkyl, C₁₋₃- (per) haloalkyl, C₁₋₃-alkoxy, C₁₋₃- (per) haloalkoxy, and OH, and
R³ and R⁴ together form a group selected from OC(R')₂O; or
R¹, R⁴, and R⁵ are each independently selected from the group consisting of H, halogen, C₁₋₄-alkyl, C₁₋₃- (per) haloalkyl, C₁₋₃-alkoxy, C₁₋₃- (per) haloalkoxy, and OH, and
R² and R³ together form a group selected from OC(R')₂O;
R⁶ and R⁷ are each independently selected from the group consisting of H, methyl, and ethyl;
R⁸ is selected from the group consisting of H, methyl, ethyl, CH₂Ph(*o*-OMe), CH₂Ph(*o*-OH), C(O)CH₂NH₂, C(O)CH(NH₂)(CH₂)₄NH₂, C(O)CH(NH₂)(CH₂)₃N(H)C(NH)(NH₂), 5-(1,2-dithiolan-3-yl)valeryl, and 2-cyano-1-phenylethyl;
R⁹ is selected from the group consisting of CO₂R¹⁰, and COSR¹⁰;
R¹⁰ is selected from the group consisting of H, methyl, and ethyl; and
each R' is independently selected from the group consisting of H, and F;
or a stereoisomer or a pharmaceutically acceptable salt thereof.

The present disclosure also provides pharmaceutical compositions comprising an effective amount of one or more compounds, or a stereoisomer or a pharmaceutically acceptable salt thereof, as defined in the present disclosure, together with one or more pharmaceutically acceptable excipient(s).

The present disclosure also provides a compound of formula (I), or a stereoisomer or pharmaceutically acceptable salt thereof, for use as a medicament.

The present disclosure also provides a compound of formula (I), or a stereoisomer or pharmaceutically acceptable salt thereof, for use in the treatment or prevention of drug addiction or a CNS related disease or condition.

Further, the present disclosure provides a compound of formula (I), or a stereoisomer or pharmaceutically acceptable salt thereof, for use in the treatment or prevention of drug addiction or a CNS related disease or condition, wherein the drug addiction or the CNS related disease or condition is selected from the group consisting of stimulant addiction, ADHD, ADD, sluggish cognitive tempo, concentration deficit disorder, motivational or reward system dysfunction, autism spectrum disorder, disruptive, impulse control, and conduct disorders, anxiety disorders, eating disorders, depression, dysthymia, Alzheimer's disease, Parkinson's disease, hyperactivity, narcolepsy, and alcoholism.

Further, the present disclosure provides methods for the preparation of compounds of formula (I), or pharmaceutically acceptable salts or a stereoisomers thereof.

The objects of the invention are achieved by compounds and methods for the preparation thereof, and said compounds for use as a medicament that are characterized by what is stated in the independent claims. The preferred embodiments of the invention are disclosed in the dependent claims.

### DETAILED DESCRIPTION

Compounds as disclosed herein and hereafter are derivatives of 3,3-disubstituted azetidines that provide the inventive properties of the compounds of the present invention. Specific substitution patterns of the compounds are beneficial for binding affinity for proteins that are associated with drug addiction or a CNS related disease or condition.

"Comprises" or "comprising" denotes that the subsequently described feature(s) or act(s) may but need not include other feature(s) or act(s). It will further be understood that reference to 'an' item refers to one or more of those items.

"Optional" or "optionally" denotes that the subsequently described event or circumstance may but need not occur, and that the description includes instances where the event or circumstance occurs and instances in which it does not.

The term "halogen" as used herein and hereafter by itself or as part of other groups refers to the Group VIIa elements and includes F, Cl, Br and I groups.

The term "alkyl" as used herein and hereafter is an aliphatic linear, branched or cyclic, especially linear or branched, hydrocarbon group having the indicated number of carbon atoms, for example C₁₋₆-alkyl has 1 to 6 carbon atoms in the alkyl moiety and thus, for example, C₁₋₄-alkyl includes methyl, ethyl, n-propyl, isopropyl, n-butyl, sec-butyl, isobutyl, tert-butyl and C₁₋₆-alkyl additionally includes, but is not limited to, branched and straight chain pentyl and hexyl. Other examples of alkyl are benzyl (may be a protecting group), 2-methoxybenzyl (CH₂Ph(*o*-OMe)), 2-hydroxybenzyl (CH₂Ph(*o*-OH)), and 2-cyano-1-phenylethyl.

The term "C₁₋₃-(per)haloalkyl" as used herein and hereafter refers to any of the above alkyl groups where one or more hydrogen atoms are replaced by halogen(s): in particular I, Br, F or Cl. Examples of haloalkyl groups include without limitation chloromethyl, fluoromethyl and -CH₂CF₃. The term "perhaloalkyl" is understood to refer to an alkyl group, in which all the hydrogen atoms are replaced by halogen atoms. Examples of perhaloalkyls include, but are not limited to, trifluoromethyl (-CF₃) and trichloromethyl (-CCl₃).

The term "C₁₋₃-alkoxy" as used herein and hereafter refers to a -O-(C₁₋₃-alkyl) group where the "C₁₋₃-alkyl" has the above-defined meaning. Examples of alkoxy groups include, but are not limited to, methoxy, ethoxy, and iso-propyloxy.

The term "C₁₋₃-(per)haloalkoxy" as used herein and hereafter refers to a -O-(C₁₋₃-(per)haloalkyl) group where the C₁₋₃-(per)haloalkyl has the above-defined meaning. Examples of (per)haloalkoxy groups include, but are not limited to, trifluoromethoxy, 2,2,2-trichloromethoxy, and 1,1,1,3,3,3-hexafluoro-isopropoxy.

The term "stereoisomer" as used herein and hereafter refers to stereoisomers of compounds. Examples of stereoisomers include, but are not limited to, enantiomers, diastereomers, cis-trans-isomers, and E-Z-isomers.

The term "pharmaceutically acceptable salt" as used herein and hereafter refers to salts which are known to be non-toxic and are commonly used in the pharmaceutical literature. Typically, these are acid addition salts or base addition salts of the referred compounds as disclosed herein and hereafter.

The expression "acid addition salt" includes any non-toxic organic and inorganic acid addition salts that that the compounds of formula (I) can form. Illustrative inorganic acids, which form suitable acid addition salts, include, but are not limited to, hydrogen chloride, hydrogen bromide, sulphuric and phosphoric acids. Illustrative organic acids, which form suitable acid addition salts, include, but are not limited to, acetic acid, lactic acid, malonic acid, succinic acid, glutaric acid, fumaric acid, malic acid, tartaric acid, citric acid, ascorbic acid, maleic acid, benzoic acid, phenylacetic acid, cinnamic acid, methane sulfonic acid, salicylic acid, and the like. The term "acid addition salt" as used herein also comprises solvates which the compounds and salts thereof are able to form, such as, for example, hydrates, alcoholates, and the like. These salts also include salts useful for the chiral resolution of racemates.

The expression "base addition salt" includes any non-toxic base addition salts that the compounds of formula (I) can form. Suitable base addition salts include, but are not limited to, those derived from inorganic bases such as aluminum, ammonium, calcium, copper, iron, lithium, magnesium, manganese, potassium, sodium, and zinc salts, in particular sodium and ammonium salts. Further examples of organic base addition salts include salts of trialkylamines, such as triethyl amine and trimethyl amine, other salts of organic amines such as methylamine, dimethylamine, trimethylamine, ethylamine, triethylamine, morpholine, and the like, and choline salts. The term "base addition salt" as used herein also comprises solvates which the compounds and salts thereof are able to form, such as, for example, hydrates, alcoholates, and the like. These salts also include salts useful for the chiral resolution of racemates.

Pharmaceutical compositions of the present invention may be administered in an effective amount within the dosage range of about 0.1 µg/kg to about 300 mg/kg, preferably between 1.0 µg/kg to 10 mg/kg body weight. Compounds of the present invention may be administered in a single daily dose, or the total daily dosage may be administered in divided doses of two, three, four, five or six times daily.

The term "effective amount" refers to an amount of a composition or a pharmaceutical composition that confers a therapeutic effect on the treated subject. The therapeutic effect may be objective (i.e. measurable by some test or marker) or subjective (i.e. subject gives an indication of or feels an effect). Such treatment need not necessarily completely ameliorate the disease or condition. Further, such treatment or prevention can be used in conjunction with other traditional treatments for treating the disease or condition known to those skilled in the art. The effective amount will typically be determined by a physician, and depend on the disease or condition to be treated, the chosen route of administration, the actual compound administered, the age, gender, weight, and response of the individual patient, the severity of the patient's symptoms, and the like.

Skilled artisans possess the knowledge and skill in the art to enable them to select suitable pharmaceutically acceptable excipients in appropriate amounts for use in the invention. In addition, there are a number of resources that are available to the skilled artisan which describe pharmaceutically acceptable excipients and may be useful in selecting suitable pharmaceutically acceptable excipients.

Suitable pharmaceutically acceptable excipients include, but are not limited to, the following types of excipients: diluents (for example starches, mannitol), fillers (for example lactose, microcrystalline cellulose or calcium hydrogen phosphate), binders (for example pre-gelatised corn starch, polyvinylpyrrolidone or methylcellulose), additives (for example magnesium stearate, talc, silica), disintegrants (for example potato starch), lubricants (for example sodium lauryl sulphate), glidants (for example fumed silica, talc, magnesium carbonate), granulating agents (for example water, ethanol), coating agents (for example hydroxypropyl methylcellulose, gelatin, waxes, shellac, plastics, plant fibers), wetting agents (for example sorbitan monopalmitate, poloxamer 407), solvents (for example water), co-solvents (for example ethanol, propylene glycol), suspending agents (for example sorbitol, cellulose derivatives, edible hydrogenated fats), emulsifiers (for example lecithin or acacia), sweeteners (for example sucrose), flavoring agents (for example cherry, lime), flavor masking agents (for example vanilla, citrus), coloring agents (for example titanium oxide), anti-caking agents (for example silicon dioxide), humectants (for example glycerine, sorbitol), chelating agents (for example EDTA salts, histidine, aspartic acid), plasticizers (for example tributyl citrate, diethyl phthalate), viscosity increasing agents (for example methylcellulose), antioxidants (for example (ascorbic acid, cysteine), preservatives (for example methyl or propyl p-hydroxybenzoates, sorbic acid or ascorbic acid), stabilizers (for example polysorbate 20 & 80, poloxamer 407), surfactants (for example polyethylene glycol, polysorbate 80), and buffering agents (for example sodium and potassium phosphates, citrate, acetate, carbonate or glycine buffers depending on the targeted pH-range). Excipients and/or auxiliaries may facilitate processing of the active agent(s) into preparations that can be used pharmaceutically. The skilled artisan will appreciate that certain pharmaceutically acceptable excipients may serve more than one function and may serve alternative functions depending on how much of the excipient is present in the pharmaceutical composition and what other ingredients are present in the pharmaceutical composition.

Pharmaceutical compositions of the invention are most preferably used alone or in combination i.e. administered simultaneously, separately, or sequentially with other active ingredients, e.g. pharmaceutically active compounds or biologic products. The amounts of the pharmaceutical composition(s) of the invention, particularly a pharmaceutical composition comprising a compound of formula (I), or stereoisomers or pharmaceutically acceptable salts thereof, and the other active ingredient(s) and the relative timings of administration will be selected in order to achieve the desired combined therapeutic effect. Pharmaceutical compositions of the invention may be administered by various routes, for example, parenteral, subcutaneous, intravenous, intra-articular, intrathecal, intramuscular, intraperitoneal, topical, and by intradermal injections, and via transdermal, rectal, buccal, oromucosal, nasal, ocular routes and via inhalation and via implant.

Pharmaceutical compositions may be formulated into suitable pharmaceutical formulations; suitable pharmaceutical formulations include, for example, solutions, dispersions, suspensions, sterile aqueous or non-aqueous solvents, emulsions, powders, capsules, tablets, pills, controlled release capsules, controlled release tablets and controlled release pills.

In addition, or alternatively, to pharmaceutically acceptable excipient (s) and/or other active ingredients(s), the pharmaceutical compositions may be combined with one or more pharmaceutically acceptable carrier(s). The term "pharmaceutically acceptable carrier (s)" as used herein and hereafter refers to substrates comprised in pharmaceutical compositions for drug delivery, which serves to improve the selectivity, effectiveness, and/or safety of drug administration. Examples of pharmaceutically acceptable carriers include, but are not limited to, pharmaceutically acceptable excipients, liposomes, (polymeric) micelles, microspheres, nanoparticles, and protein-drug conjugates.

The pharmaceutical compositions of the invention are prepared using techniques and methods known to those skilled in the art.

Pharmaceutical formulations of the pharmaceutical compositions as disclosed herein and hereafter for parenteral or topical use may include, but are not limited to, sterile aqueous and/or non-aqueous solvents, suspensions, and emulsions.

Examples of non-aqueous solvents of pharmaceutical formulations are propylene glycol, polyethylene glycol, vegetable oil, fish oil, and injectable organic esters. Aqueous carriers include, but are not limited to, water, water-alcohol solutions, including saline and buffered medial parenteral vehicles including sodium chloride solution, Ringer's dextrose solution, dextrose plus sodium chloride solution, Ringer's solution containing lactose, or fixed oils. Intravenous vehicles include, but are not limited to, fluid and nutrient replenishers, electrolyte replenishers, such as those based on Ringer's dextrose and the like. Aqueous pharmaceutical compositions according to the invention may comprise suitable buffer agents, such as sodium and potassium phosphates, citrate, acetate, carbonate or glycine buffers depending on the targeted pH-range. The use of sodium chloride as a tonicity adjuster is also useful. Pharmaceutical compositions may include other excipients, such as stabilizing agents or preservatives. Useful stabilizing excipients include surfactants (polysorbate 20 & 80, poloxamer 407), polymers (polyethylene glycols, povidones), carbohydrates (sucrose, mannitol, glucose, lactose), alcohols (sorbitol, glycerol propylene glycol, ethylene glycol), suitable proteins (albumin), suitable amino acids (glycine, glutamic acid), fatty acids (ethanolamine), antioxidants (ascorbic acid, cysteine etc.), chelating agents (EDTA salts, histidine, aspartic acid) or metal ions (Ca, Ni, Mg, Mn). Among useful preservative agents are benzyl alcohol, chlorobutanol, benzalkonium chloride and possibly parabens. The pharmaceutical composition according to the present invention may be provided in concentrated form or in form of a powder to be reconstituted on demand. In such cases formulations of powder for solution for injection/infusion excipients mentioned above may be used. In case of lyophilizing, certain cryoprotectants are preferred, including polymers (povidones, polyethylene glycol, dextran), sugars (sucrose, glucose, lactose), amino acids (glycine, arginine, glutamic acid) and albumin. If solution for reconstitution is added to the packaging, it may consist e.g., of pure water for injection or sodium chloride solution or dextrose or glucose solutions.

The terms "treatment or prevention" as used herein and hereafter includes prophylaxis, or prevention of, as well as lowering the individual's risk of falling ill with the named disorder or condition, or alleviation, amelioration, elimination, or cure of the said disorder or condition once it has been established.

The terms "administering" or "administered" to a subject or patient includes dispensing, delivering or applying a compound as disclosed herein and hereafter, or a stereoisomer or a pharmaceutically acceptable salt thereof, or a pharmaceutical composition as disclosed herein and hereafter to the subject or patient by any suitable route for delivery of the composition or pharmaceutical composition to a site in the body where desired.

Compounds of formula (I) of the present disclosure may be useful in therapy, especially in the treatment or prevention of drug addiction or a CNS related disease or condition in animals, in particular mammals, and humans. In particular, compounds of formula (I) possess pharmacological properties for the treatment and/or prophylaxis of drug addiction or a CNS related disease or condition that include, but are not limited to, stimulant addiction, ADHD, ADD, sluggish cognitive tempo, concentration deficit disorder, motivational or reward system dysfunction, autism spectrum disorder, disruptive, impulse control, and conduct disorders, anxiety disorders, eating disorders, depression, dysthymia, Alzheimer's disease, Parkinson's disease, anxiety, hyperactivity, narcolepsy, and alcoholism.

The compounds of formula (I) of the present disclosure may bind to one or more protein involved in drug addiction or a CNS related disease or condition, wherein examples of the proteins include, but are not limited to, trace amine receptors (trace amine-associated receptors (TAARs), such as TAAR1), dopamine- and serotonin receptors, dopamine- and serotonin transporters, acyl and methyl transferases, norpinephrine transporter, monoamino-oxidases such as MAO-A, catecholine-O-methylransferase (COMT), adrenergic receptors, tyrosine hydroxylase, histamine receptors such as H1 histamine receptor, orexin receptors, NMDA-receptors, sigma-1 receptor, muscarinic and nicotinic acetylcholine receptors such as M1, M2, M3, and M4 muscarinic acetylcholine receptors, opioid receptors such as µ-opioid receptors (MOR), neuropeptide receptors such as Neuropeptide Y2 receptor, melanocortin receptors (excluding MC3R), neurokinin receptors such as NKR2 and NK3R, corticotropin-releasing factor receptor 1, acetylcholinesterase, 1B melatonin receptor, cholinergic receptors, and dopamine beta-hydroxylase (dopamine beta-monooxygenase, DBH), to name a few.

The compounds of formula (I) as disclosed herein and hereafter may bind to and inhibit a protein including, but not limited to, HIV-1 reverse transcriptase, oxidosqualene cyclase, aldose reductase, and hepatitis C virus NS5B RNA-dependent RNA polymerase.

The term "protecting group" as used herein and hereafter refers to a chemical group, which is covalently attached to an atom or a functional group, which has been modified by the protecting group. Said protecting group may enable chemoselectivity in a reaction, therefore, the protecting group may protect an atom or a functional group from, at least partially, reacting in a reaction. It is to be understood that the protecting group protects the atom or functional group fully or partly, i.e., the atom or the functional group protected with a protecting group may or may not react partly in a reaction. Skilled artisans possess the knowledge and skill in the art to enable them to select suitable protecting groups for the atoms or functional groups to be protected. In addition, there are a number of resources that are available to the skilled artisan which describe protecting groups and may be useful in selecting suitable protecting groups for the atoms or functional groups to be protected. For suitable protecting groups and methods to protect compounds with suitable protecting groups, see, for example, Protective Groups in Organic Synthesis, 4th Edition, 2007, John Wiley & Sons, Inc., Hoboken, New Jersey. Examples of atoms and functional groups that may be protected with protecting groups include, but are not limited to, O, S, N, NH, NH₂, OH, SH, carbonyls such as aldehydes and ketones, ethers, esters, and amides. Examples of protecting groups include, but are not limited to, carbobenzyloxy (Cbz), p-methoxybenzyl carbonyl, BOC, Fmoc, acetyl, benzoyl, phenyl, benzyl, trityl, sulfonyls such as phenylsulfonyl, tosyl (Ts), mesyl, and trifyl; tosylate, silyl ethers such as trimethylsilyl (TMS), tert-butyldimethylsilyl (TBDMS), tri-iso-propylsilyloxymethyl (TOM), and triisopropylsilyl (TIPS) ethers; tetrahydropyranyl (THP), p-methoxyphenyl ether (PMP), p-methoxybenzyl ether (PMB), β-methoxyethoxymethyl ether (MEM), pivaloyl, thioethers, acetals, ketals, dithianes, benzyl esters, *tert*-butyl esters, orthoesters, and photolabile groups.

The term "activating group" as used herein and hereafter refers to a functional group of a chemical compound that promotes a reaction to occur and/or has a positive influence of the overall reaction rate and/or have a directing effect on positional isomer of the products that are formed. Said activating group may or may not be part of the formed product, i.e. it is to be understood that the activating group may be present in the product, or the activating group may be a leaving group, or part of the leaving group, in e.g. S_{N}2, S_{N}1 and addition-elimination reactions. A compound disclosed herein may have one or more activating group(s) that may be the same or different. Examples of activating groups include, but are not limited to, sulfonyls such as phenylsulfonyl, tosyl (Ts), mesyl, and trifyl; halogens such as Cl, Br, I, and F; (substituted) amino groups such as 1-imidazolyl, ion pair of intermediates formed from the reaction between PPh₃, azodicarboxylates, such as DEAD and DIAD, and carboxylic acid, amides, esters such as O-acylisourea of DCC and carboxyl acid, hydroxy, alkoxy such as *1H*-1,2,3-benzotriazol-1-olate, anion of *N*-hydroxysuccinimide, acyloxy, phenyliodine(III) diacetate [PhI(OAc)₂, (PIDA)], metals and complexes thereof such as Ni, Ru, Os, Ir, Rh, Pd, Fe-PNP; thiols, alkyls, (per)haloalkoxy, (per)haloalkyl, and photolabile groups.

The term "activating group reactant" as used herein and hereafter refers to a chemical compound, comprising an activating group, wherein the chemical compound reacts with another chemical compound in a reaction to form a chemical compound or an intermediate comprising said activating group. It is to be understand that there may be different activating group reactants comprising the same activating group. A reaction may comprise one or more activating group reactant(s) that may be the same or different. Examples of activating group reactants include, but are not limited to, 1,1'-carbonyldiimidazole (CDI), diethyl azodicarboxylate (DEAD), diisopropyl azodicarboxylate (DIAD), *N,N'-*dicyclohexylcarbodiimide (DCC), triphenylphosphine (PPh₃), phenyliodine(III) diacetate [PhI(OAc)₂, (PIDA)], sulfinic acids such as phenylsulfinic acid and *p-*toluenesulfinic acid; mesyl halides such as methanesulfonyl chloride; trifyl azide, trifluoromethanesulfonyl chloride, halogens such as Br₂; metal halides such as NaI; trifluoroacetyl chloride, and trifluoroacetic anhydride.

The term "activating agent" as used herein and hereafter refers to a substance or chemical compound added to a reaction to facilitate a chemical reaction. Said activating agent may or may not be a catalyst. It is to be understood that said activating agent may or may not be consumed in the reaction. Examples of activating agents include, but are not limited to, acids such as HCl, and AcOH; bases such as NaOH and NaH; water, 4-dimethylaminopyridine (DMAP), triphenylphosphine (PPh₃), metal halides such as NaI; Lewis acids such as TiCl₄, boron trifluoride, and boron trifluoride diethyl etherate.

The terms "performing one or more reactions" as used herein and hereafter refers to one or more method steps, i.e., reacting a compound of a method for the preparation of a compound of formula (I) in an indicated reaction. It is to be understood that if e.g. two reactions are performed a first reaction may be performed forming one or more first reaction products, wherein at least one of the first reaction products may be used in the subsequent reactions forming one or more second reaction products that may or may not be the compound of formula (I). Further, the one or more (first, second, third, fourth, and/or fifth) reaction products may or may not be separated and/or purified after the (first, second, third, fourth, and/or fifth) reaction, i.e., the reactions product may or may not be used directly in subsequent reaction. In addition, one or more reactions may be performed simultaneously as long as the reactions conditions allows it.

The term "esterification" as used herein and hereafter refers to a reaction wherein an ester or a thioester is formed from a carboxylic acid as disclosed herein and hereafter.

The term "transesterification" as used herein and hereafter refers to a reaction wherein an alkoxy group of an ester or a thiolate group of a thioester is converted to another alkoxy group or thiolate group, i.e., an ester group or thioester group of a compound disclosed herein and hereafter is changed to another ester group or thioester group. Skilled artisans possess the knowledge and skill in the art to enable them to select suitable reactants and reagents for the transesterification. Examples of transesterification reactions include, but are not limited to, combining HCl, methanol or ethanol or methanethiol or ethanethiol, and a compound of a method for the preparation of a compound of formula (I) to form a first composition, and optionally heating the formed first composition.

The term "amidation" as used herein and hereafter refers to a reaction, wherein an ester group, a carboxylic acid group, and/or an amine group of a compound of a method for the preparation of a compound of formula (I) is converted to an amide group. I.e., the carboxylic group, the ester group, and/or the amine group of a compound of a method for the preparation of a compound of formula (I) is reacting forming an amide group of said compound. Preferably, the amine group of an azetidine as disclosed herein and hereafter is converted to an amide group. Examples of amidation reactions include, but are not limited to, combining a carboxylic acid and a compound of a method for the preparation of a compound of formula (I), preferably an azetidine, optionally in the presence of one or more activating group reactant(s) and/or one or more activating agent(s), to form a first composition, and optionally heating the formed first composition to form an amide.

The term "reductive alkylation" as used herein and hereafter refers to reductive amination, wherein a carbonyl group of a chemical compound is converted to an amine via an intermediate imine. Examples of reductive alkylation reactions include, but are not limited to, combining methanal or ethanal with a compound comprising a secondary amine group of a method for the preparation of a compound of formula (I) (i.e., for example a compound of formula (I), wherein R⁸ is H, and R¹⁰ is methyl or ethyl), optionally in the presence of one or more water removing compounds or apparatus arranged to remove water, forming an imine that is combined with a reducing agent, such as sodium borohydride, to form a tertiary amine.

The term "N-alkylation" as used herein and hereafter refers to a reaction, wherein an amine group is alkylated. Examples of N-alkylation reactions include, but are not limited to, combining a haloalkane such as methylbromide, ethylbromide, methyliodide, and ethyliodide, and a secondary amine group comprising compound of a method for the preparation of a compound of formula (I), optionally in the presence of one or more activating group reactant(s) and/or one or more activating agent(s) such as sodium iodide, to form a N-alkylated compound.

The term "reduction" as used herein and hereafter refers to a reaction, wherein a functional group of a chemical compound is reduced, i.e., the oxidation state of a first functional group is reduced thereby forming a second functional group with a lower oxidation state compared to the oxidation state of the first functional group.

The term "hydrolysis" as used herein and hereafter refers to a chemical reaction in which a molecule of water breaks one or more chemical bonds. Typically, hydrolysis is performed on a carboxylic ester forming the respective carboxylic acid, or on an amide forming the respective carboxylic acid.

The term "*O*-alkylation" as used herein and hereafter refers to a reaction, wherein an OH group is alkylated. Examples of *O*-alkylation reactions include, but are not limited to, combining a haloalkane such as methylbromide, ethylbromide, methyliodide, and ethyliodide, and a hydroxy group comprising compound of a method for the preparation of a compound of formula (I), optionally in the presence of one or more bases, one or more activating group reactant(s) and/or one or more activating agent(s) such as sodium iodide, to form a *O*-alkylated compound.

The term "reductive trifluoromethylation" as used herein and hereafter refers to a chemical reaction that entails the introduction of one or more CF₃ into a compound by also reducing the oxidation state of said compound. An example of reductive trifluoromethylation include, but is not limited to, the transformation of a C(O)NHMe group of a compound to a CH(CF₃)NHMe group of the compound, see, for example, Chen et al., Chemoselective direct reductive trifluoromethylation of amides: a flexible access to functionalized α-trifluoromethylamines, Org. Chem. Front., 2018,5, 943-947. Particularly, a compound having one or more CF₃ groups has enhanced metabolic stability, lipophilicity, and bioavailability.

The term "fluorination" as used herein and hereafter refers to a chemical reaction that entails the introduction of one or more F into a compound.

The terms "performing one or more deprotection reaction(s)" as used herein and hereafter refers to performing one or more reaction (s), wherein a protecting group is removed from a compound. Skilled artisans possess the knowledge and skill in the art to enable them to select suitable reactants or reagents for the deprotection reaction(s). In addition, there are a number of resources that are available to the skilled artisan which describe suitable reagents and reactants and may be useful in selecting suitable reagents and reactants for the protecting groups to be deprotected. For suitable deprotecting reactions, reactants and reagents, see, for example, Protective Groups in Organic Synthesis, 4th Edition, 2007, John Wiley & Sons, Inc., Hoboken, New Jersey. Examples of reactants and reagents usable in the deprotection reaction(s) (with examples of the protecting group to be removed in parenthesis) include, but are not limited to, tetra-*n*-butylammonium fluoride (TMS), H₂ (benzyl), bases such as NaOH (acetyl), acids such as pyridinium *p*-toluenesulfonate and EtOH (THP), and 2,3-dichloro-5,6-dicyano-*p-*benzoquinone (PMB).

The compounds of the invention are disclosed in claim 1 and are part of the embodiments as described in the following paragraphs:
In one aspect is disclosed a compound of formula (I) 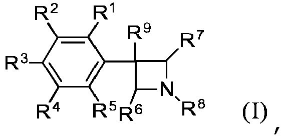 wherein
R¹, R², R³, R⁴, and R⁵ are each independently selected from the group consisting of H, halogen, C₁₋₄-alkyl, C₁₋₃- (per) haloalkyl, C₁₋₃-alkoxy, C₁₋₃- (per) haloalkoxy, and OH; or
R¹, R², and R⁵ are each independently selected from the group consisting of H, halogen, C₁₋₄-alkyl, C₁₋₃- (per) haloalkyl, C₁₋₃-alkoxy, C₁₋₃- (per) haloalkoxy, and OH, and
R³ and R⁴ together form a group selected from OC(R')₂O; or
R¹, R⁴, and R⁵ are each independently selected from the group consisting of H, halogen, C₁₋₄-alkyl, C₁₋₃- (per) haloalkyl, C₁₋₃-alkoxy, C₁₋₃- (per) haloalkoxy, and OH, and
R² and R³ together form a group selected from OC(R')₂O;
R⁶ and R⁷ are each independently selected from the group consisting of H, methyl, and ethyl;
R⁸ is selected from the group consisting of H, methyl, ethyl, CH₂Ph(*o*-OMe), CH₂Ph(*o*-OH), C(O)CH₂NH₂, C(O)CH(NH₂)(CH₂)₄NH₂, C(O)CH(NH₂)(CH₂)₃N(H)C(NH)(NH₂), 5-(1,2-dithiolan-3-yl)valeryl, and 2-cyano-1-phenylethyl;
R⁹ is selected from the group consisting of CO₂R¹⁰, and COSR¹⁰;
R¹⁰ is selected from the group consisting of H, methyl, and ethyl; and
each R' is independently selected from the group consisting of H, and F;
or a stereoisomer or a pharmaceutically acceptable salt thereof.

These compounds may have a particular strong binding affinity to one or more proteins that are associated with drug addiction or a CNS related disease or condition.

Additionally, or alternatively, in embodiments R⁹ is selected from the group consisting of CO₂R¹⁰, COSR¹⁰, CH₂OR¹⁰', C(O)CH₂R¹⁰', CH(CF₃)NHR¹⁰', C(O)NFR¹⁰', and C(O)N(R¹⁰')₂;
R¹⁰ is selected from the group consisting of H, methyl, and ethyl; and
each R¹⁰' is independently selected from the group consisting of H, methyl, and ethyl;
or a stereoisomer or a pharmaceutically acceptable salt thereof.

Additionally, or alternatively, in embodiments R⁹ is selected from the group consisting of CO₂R¹⁰; and R¹⁰ is as defined in the present disclosure; or a stereoisomer or a pharmaceutically acceptable salt thereof. These compounds may have a particular strong binding affinity to proteins that are associated with drug addiction or a CNS related disease or condition.

Additionally, or alternatively, in embodiments R¹, R² and R⁵ are H; R³ and R⁴ are each independently selected from the group consisting of H, halogen, C₁₋₄-alkyl; R⁹ is CO₂R¹⁰; and R¹⁰ is as defined in the present disclosure; or a stereoisomer or a pharmaceutically acceptable salt thereof. These compounds may have a particular strong binding affinity to proteins that are associated with drug addiction or a CNS related disease or condition.

Additionally, or alternatively, in embodiments at least one of R⁶, R⁷, and R⁸ is each independently selected from the group consisting of methyl, and ethyl, and the other ones of R⁶, R⁷, and R⁸ are each independently selected from the group consisting of H, methyl, and ethyl; or a stereoisomer or a pharmaceutically acceptable salt thereof. These compounds may have a particular strong binding affinity to proteins that are associated with drug addiction or a CNS related disease or condition.

Additionally, or alternatively, in embodiments R¹, `R² and R⁵ are H; and R³ and R⁴ are both halogen, preferably F; or one of R³ and R⁴ is halogen or methyl, preferably F, and the other one of R³ and R⁴ is H provided that R⁸ is selected from the group consisting of methyl, ethyl, CH₂Ph(*o*-OMe), CH₂Ph(*o*-OH), C(O)CH₂NH₂, C(O)CH(NH₂)(CH₂)₄NH₂, C(O)CH(NH₂)(CH₂)₃N(H)C(NH)(NH₂), 5-(1,2-dithiolan-3-yl)valeryl, and 2-cyano-1-phenylethyl, or provided that at least one of R⁶, and R⁷ is each independently selected from the group consisting of methyl, and ethyl, and the other one of R⁶, and R⁷ is selected from the group consisting of H, methyl, and ethyl; or a stereoisomer or a pharmaceutically acceptable salt thereof. These compounds may have a particular strong binding affinity to proteins that are associated with drug addiction or a CNS related disease or condition.

Additionally, or alternatively, in embodiments R¹, R² and R⁵ are H; and R³ and R⁴ are both halogen, preferably F; or one of R³ and R⁴ is halogen or methyl, preferably F, and the other one of R³ and R⁴ is H provided that R⁸ is selected from the group consisting of methyl, ethyl, CH₂Ph(*o*-OMe), CH₂Ph(*o*-OH), C(O)CH₂NH₂, C(O)CH(NH₂)(CH₂)₄NH₂, C(O)CH(NH₂)(CH₂)₃N(H)C(NH)(NH₂), 5-(1,2-dithiolan-3-yl)valeryl, and 2-cyano-1-phenylethyl, preferably selected from the group consisting of methyl, and ethyl; or a stereoisomer or a pharmaceutically acceptable salt thereof. These compounds may have a particular strong binding affinity to proteins that are associated with drug addiction or a CNS related disease or condition.

Additionally, or alternatively, in embodiments R¹, R² and R⁵ are H; and R³ and R⁴ are both halogen, preferably F; or one of R³ and R⁴ is halogen or methyl, preferably F, and the other one of R³ and R⁴ is H provided that at least one of R⁶, and R⁷ is each independently selected from the group consisting of methyl, and ethyl, and the other one of R⁶, and R⁷ is selected from the group consisting of H, methyl, and ethyl; or a stereoisomer or a pharmaceutically acceptable salt thereof. These compounds may have a particular strong binding affinity to proteins that are associated with drug addiction or a CNS related disease or condition.

Additionally, or alternatively, in embodiments R¹, R² and R⁵ are H; and R⁸ is selected from the group consisting of methyl, ethyl, CH₂Ph(*o*-OMe), CH₂Ph(*o*-OH), C(O)CH₂NH₂, C(O)CH(NH₂)(CH₂)₄NH₂, C(O)CH(NH₂)(CH₂)₃ N(H)C(NH)(NH₂), 5- (1, 2-dithiolan-3-yl)valeryl, and 2-cyano-1-phenylethyl; or a stereoisomer or a pharmaceutically acceptable salt thereof. These compounds may have a particular strong binding affinity to proteins that are associated with drug addiction or a CNS related disease or condition.

Additionally, or alternatively, in embodiments R¹, R² and R⁵ are H; at least one of R³ and R⁴ is each independently selected from the group consisting of halogen, C₁₋₄-alkyl, C₁₋₃- (per) haloalkyl, C₁₋₃-alkoxy, C₁₋₃-(per)haloalkoxy, and OH, preferably selected from the group consisting of F, Cl, methyl, and ethyl, and the other one of R³, and R⁴ is selected from the group consisting of H, halogen, C₁₋₄-alkyl, C₁₋₃-(per) haloalkyl, C₁₋₃-alkoxy, C₁₋₃- (per) haloalkoxy, and OH, preferably selected from the group consisting of H, F, Cl, methyl, and ethyl; and R⁸ is selected from the group consisting of H, methyl, ethyl, CH₂Ph(*o*-OMe), CH₂Ph(*o*-OH), C(O)CH₂NH₂, C(O)CH(NH₂)(CH₂)₄NH₂, C(O)CH(NH₂)(CH₂)₃ N(H)C(NH)(NH₂), 5-(1,2-dithiolan-3-yl)valeryl, and 2-cyano-1-phenylethyl, preferably selected from the group consisting of H, methyl, and ethyl; or a stereoisomer or a pharmaceutically acceptable salt thereof. These compounds may have a particular strong binding affinity to proteins that are associated with drug addiction or a CNS related disease or condition.

Additionally, or alternatively, in embodiments R⁸ is selected from the group consisting of H, methyl, ethyl, CH₂Ph(*o*-OMe), CH₂Ph(*o*-OH), C(O)CH₂NH₂, C(O)CH(NH₂)(CH₂)₄NH₂, C(O)CH(NH₂)(CH₂)₃ N(H)C(NH)(NH₂), 5-(1,2-dithiolan-3-yl)valeryl, and 2-cyano-1-phenylethyl, provided that if R⁸ is H then at least one of R⁶, and R⁷ is each independently selected from the group consisting of methyl, and ethyl, and the other one of R⁶, and R⁷ is selected from the group consisting of methyl, and ethyl; or a stereoisomer or a pharmaceutically acceptable salt thereof. These compounds may have a particular strong binding affinity to proteins that are associated with drug addiction or a CNS related disease or condition.

Additionally, or alternatively, in embodiments R¹, R² and R⁵ are H; and R³ and R⁴ are each independently selected from the group consisting of H, halogen, C₁₋₄-alkyl, C₁₋₃- (per) haloalkyl, C₁₋₃-alkoxy, C₁₋₃- (per) haloalkoxy, and OH, with the provision that if one of R³ or R⁴ is H then at least one of R⁶, and R⁷ is each independently selected from the group consisting of methyl, and ethyl, and the other one of R⁶, and R⁷ is selected from the group consisting of H, methyl, and ethyl, or R⁶, and R⁷ are both H, and R⁸ is selected from the group consisting of methyl, ethyl, CH₂Ph(*o*-OMe), CH₂Ph(*o*-OH), C(O)CH₂NH₂, C(O)CH(NH₂)(CH₂)₄NH₂, C(O)CH(NH₂)(CH₂)₃ N (H) C (NH) (NH₂), 5-(1,2-dithiolan-3-yl)valeryl, and 2-cyano-1-phenylethyl, preferably selected from the group consisting of methyl, and ethyl; or a stereoisomer or a pharmaceutically acceptable salt thereof. These compounds may have a particular strong binding affinity to proteins that are associated with drug addiction or a CNS related disease or condition.

Additionally, or alternatively, in embodiments at least one of R⁶, R⁷, and R⁸ is each independently selected from the group consisting of methyl, and ethyl, and the other ones of R⁶, R⁷, and R⁸ are each independently selected from the group consisting of H, methyl, and ethyl; R⁹ is CO₂R¹⁰; and R¹⁰ is selected from the group consisting of methyl, and ethyl; or a stereoisomer or a pharmaceutically acceptable salt thereof. These compounds may have a particular strong binding affinity to one or more proteins that are associated with drug addiction or a CNS related disease or condition, such as trace amine receptor 1 (TAAR1), norepinephrine transporter (NET), 5-HT2A receptor, 5-HT2B receptor, OX1 orexin receptor, Mu-opioid receptor, Neurokinin 1 receptor (NK1R), Melanocortin 3 receptor (MC3R), dopamine transporter (DAT), phenylethanolamine N-methyltransferase (PNMT), dopamine 4 (D4) receptor, N-acetylserotonin methyltransferase, 1A Melatonin receptor, Melanocortin 1 receptor (MSHR, MC1R), Alpha-2A adrenergic receptor, Prolactin receptor, dopamine 3 (D3) receptor, Kappa-opioid receptor, serotonin transporter (SERT), 5-HT1A receptor, 5-HT1B receptor, β1 adrenergic receptor, 5-HT2C receptor, Alpha-1B adrenergic receptor, Alpha-2B adrenergic receptor, Melanocortin 2 receptor (ACTHR, MC2R), Melanocortin 4 receptor (MC4R), 5-HT3 receptor, COMT, Alpha-1A adrenergic receptor, Nociceptin / orphanin receptor, β2 adrenergic receptor, Phenylalanine hydroxylase, Tyrosine hydroxylase, H1 histamine receptor, H3 histamine receptor, Neurokinin 2 receptor (NK2R), OX2 orexin receptor, NMDA receptor GluN1, NMDA receptor Glu2B, M2 muscarinic acetylcholine receptor, neuropeptide Y1 receptor, Delta-opioid receptor, M1 muscarinic acetylcholine receptor, M3 Muscarinic acetylcholine receptor, M4 muscarinic acetylcholine, Acetylcholinesterase, Melanocortin 5 receptor (MC5R), Neuropeptide Y2 receptor, Neurokinin 3 receptor (NK3R), Oxytocin receptor (OXYR), and Corticotropin-releasing factor receptor 1 (CRF1R).

Additionally, or alternatively, in embodiments R⁶ and R⁷ are each independently selected from the group consisting of methyl, and ethyl, or one of R⁶ and R⁷ is selected from the group consisting of methyl, and ethyl, and the other one of R⁶ and R⁷ is H; R⁸ is selected from the group consisting of H, methyl, and ethyl; R⁹ is CO₂R¹⁰; and R¹⁰ is selected from the group consisting of methyl, and ethyl; or a stereoisomer or a pharmaceutically acceptable salt thereof.

Additionally, or alternatively, in embodiments R⁶ and R⁷ are both H; R⁹ is CO₂R¹⁰; and R¹⁰ is selected from the group consisting of methyl, and ethyl; or a stereoisomer or a pharmaceutically acceptable salt thereof. These compounds may have a particular strong binding affinity to one or more proteins that are associated with drug addiction or a CNS related disease or condition, such as dopamine transporter (DAT), MAO-B, dopamine 2 (D2) receptor, MAO-A, Alpha-2C adrenergic receptor, α4β2 nicotinic receptor, CHK1 kinase, serotonin transporter (SERT), serotonin N-acetyltransferase, H1 histamine receptor, Neuropeptide Y2 receptor, and Oxytocin receptor (OXYR). Additionally, in embodiments R³ is methyl or methoxy; and R¹, R², R³, R⁴, R⁵, and R⁸ is H. These compounds may have a particular strong binding affinity to one or more of dopamine 2 (D2) receptor, MAO-A, Alpha-2C adrenergic receptor, α4β2 nicotinic receptor, CHK1 kinase, and serotonin transporter (SERT).

Additionally, or alternatively, in embodiments R¹, R² and R⁵ are H; one of R³ and R⁴ is methoxy, and the other one of R³ and R⁴ is H; R⁹ is CO₂R¹⁰; and R¹⁰ is selected from the group consisting of methyl, and ethyl; or a stereoisomer or a pharmaceutically acceptable salt thereof. These compounds may have a particular strong binding affinity to one or more of serotonin transporter (SERT), 5-HT1B receptor, β1 adrenergic receptor, Phenylalanine hydroxylase, and Tyrosine hydroxylase. Additionally, in embodiments at least one of R⁶ and R⁷ is each independently selected from the group consisting of methyl, and ethyl, preferably methyl; and the other one of R⁶ and R⁷ is H, methyl, or ethyl, preferably H, or methyl; R⁸ is selected from the group consisting of methyl, and ethyl, preferably ethyl; R⁹ is CO₂R¹⁰; and R¹⁰ is selected from the group consisting of methyl, and ethyl, preferably methyl. These compounds may have a particular strong binding affinity to one or more of 5-HT1B receptor, β1 adrenergic receptor, Phenylalanine hydroxylase, and Tyrosine hydroxylase.

Additionally, or alternatively, in embodiments R¹ and R² are H; at least one of R³, R⁴, and R⁵ is F, and the other ones of R³, R⁴, and R⁵ are H; R⁶ and R⁷ are each independently selected from the group consisting of H, methyl, and ethyl; R⁹ is CO₂R¹⁰; and R¹⁰ is selected from the group consisting of methyl, and ethyl; or a stereoisomer or a pharmaceutically acceptable salt thereof. In embodiments, R¹ and R² are H; one of R³, R⁴, and R⁵ is F, and the other ones of R³, R⁴, and R⁵ are H; R⁶ and R⁷ are each independently selected from the group consisting of H, methyl, and ethyl; R⁹ is CO₂R¹⁰; and R¹⁰ is selected from the group consisting of methyl, and ethyl.

Additionally, or alternatively, in embodiments R¹, R² and R⁵ are H; one of R³ and R⁴ is selected from the group consisting of F, Cl, methyl, and ethyl, and the other one of R³ and R⁴ is selected from the group consisting of H, F, methyl, and ethyl; R⁹ is CO₂R¹⁰; and R¹⁰ is selected from the group consisting of methyl, and ethyl; or a stereoisomer or a pharmaceutically acceptable salt thereof.

Additionally, or alternatively, in embodiments R¹, R² and R⁵ are H; R³ and R⁴ are both F, or one of R³ and R⁴ is F and the other one of R³ and R⁴ is H; R⁶ and R⁷ are each independently selected from the group consisting of H, and methyl; R⁹ is CO₂R¹⁰; and R¹⁰ is selected from the group consisting of methyl, and ethyl; or a stereoisomer or a pharmaceutically acceptable salt thereof. These compounds may have a particular strong binding affinity to one or more proteins that are associated with drug addiction or a CNS related disease or condition, such as trace amine receptor 1 (TAAR1), norepinephrine transporter (NET), 5-HT2A receptor, 5-HT2B receptor, OX1 orexin receptor, Mu-opioid receptor, Neurokinin 1 receptor (NK1R), Melanocortin 3 receptor (MC3R), dopamine transporter (DAT), MAO-B, phenylethanolamine N-methyltransferase (PNMT), N-acetylserotonin methyltransferase, 1A Melatonin receptor, Melanocortin 1 receptor (MSHR, MC1R), Alpha-2A adrenergic receptor, and Prolactin receptor, dopamine 3 (D3) receptor, Kappa-opioid receptor, 5-HT3 receptor, OX2 orexin receptor, NMDA receptor GluN1, NMDA receptor Glu2B, M2 muscarinic acetylcholine receptor, neuropeptide Y1 receptor, Delta-opioid receptor, M1 muscarinic acetylcholine receptor, M3 Muscarinic acetylcholine receptor, M4 muscarinic acetylcholine receptor, and Corticotropin-releasing factor receptor 1 (CRF1R) .

Additionally, or alternatively, in embodiments R¹, R² and R⁵ are H; R³ and R⁴ are both F; R⁶ and R⁷ are each independently selected from the group consisting of H, and methyl; R⁹ is CO₂R¹⁰; and R¹⁰ is selected from the group consisting of methyl, and ethyl; or a stereoisomer or a pharmaceutically acceptable salt thereof. These compounds may have a particular strong binding affinity to one or more proteins that are associated with drug addiction or a CNS related disease or condition, such as trace amine receptor 1 (TAAR1), norepinephrine transporter (NET), 5-HT2A receptor, 5-HT2B receptor, OX1 orexin receptor, Mu-opioid receptor, Neurokinin 1 receptor (NK1R), Melanocortin 3 receptor (MC3R), dopamine transporter (DAT), MAO-B, N-acetylserotonin methyltransferase, 1A Melatonin receptor, Melanocortin 1 receptor (MSHR, MC1R), Alpha-2A adrenergic receptor, Prolactin receptor, serotonin transporter (SERT), 5-HT1A receptor, COMT, NMDA receptor GluN1, Delta-opioid receptor, M1 muscarinic acetylcholine receptor, M3 Muscarinic acetylcholine receptor, M4 muscarinic acetylcholine, Acetylcholinesterase, and Melanocortin 5 receptor (MC5R).

Additionally, or alternatively, in embodiments R¹, R² and R⁵ are H; one of R³ and R⁴ is selected from the group consisting of methyl, and ethyl, and the other one of R³ and R⁴ is H; R⁹ is CO₂R¹⁰; and R¹⁰ is selected from the group consisting of methyl, and ethyl; or a stereoisomer or a pharmaceutically acceptable salt thereof.

Additionally, or alternatively, in embodiments R¹, R² and R⁵ are H; one of R³ and R⁴ is methyl and the other one of R³ and R⁴ is H; R⁹ is CO₂R¹⁰; and R¹⁰ is selected from the group consisting of methyl, and ethyl; or a stereoisomer or a pharmaceutically acceptable salt thereof. These compounds may have a particular strong binding affinity to one or more proteins that are associated with drug addiction or a CNS related disease or condition, such as dopamine 2 (D2) receptor, MAO-A, Alpha-2C adrenergic receptor, α4β2 nicotinic receptor, CHK1 kinase, dopamine 4 (D4) receptor, dopamine 1 (D1) receptor, Sigma-1 receptor, 1B Melatonin receptor, serotonin N-acetyltransferase, 5-HT1B receptor, β1 adrenergic receptor, 5-HT2C receptor, Alpha-1B adrenergic receptor, Alpha-2B adrenergic receptor, Melanocortin 2 receptor (ACTHR, MC2R), Melanocortin 4 receptor (MC4R), COMT, Alpha-1A adrenergic receptor, Nociceptin / orphanin receptor, β2 adrenergic receptor, Tyrosine hydroxylase, H1 histamine receptor, NMDA receptor GluN1, Acetylcholinesterase, Melanocortin 5 receptor (MC5R), Neuropeptide Y2 receptor, Neuropeptide Y5 receptor, Neurokinin 3 receptor (NK3R), and Corticotropin-releasing factor receptor 1 (CRF1R).

Additionally, or alternatively, in embodiments R¹, R² and R⁵ are H; R³ and R⁴ are both F; R⁸ is selected from the group consisting of methyl, and ethyl; R⁹ is CO₂R¹⁰; and R¹⁰ is selected from the group consisting of methyl, and ethyl; or a stereoisomer or a pharmaceutically acceptable salt thereof. These compounds may have a particular strong binding affinity to one or more proteins that are associated with drug addiction or a CNS related disease or condition, such as trace amine receptor 1 (TAAR1), norepinephrine transporter (NET), 5-HT2A receptor, 5-HT2B receptor, OX1 orexin receptor, Mu-opioid receptor, Neurokinin 1 receptor (NK1R), Melanocortin 3 receptor (MC3R), dopamine transporter (DAT), N-acetylserotonin methyltransferase, 1A Melatonin receptor, Melanocortin 1 receptor (MSHR, MC1R), Alpha-2A adrenergic receptor, Prolactin receptor, OX2 orexin receptor, Delta-opioid receptor, M1 muscarinic acetylcholine receptor, M3 Muscarinic acetylcholine receptor, and M4 muscarinic acetylcholine receptor.

Additionally, or alternatively, in embodiments the compound is selected from the group consisting of:
ethyl 3-(3,4-difluorophenyl)-1,2,4-trime-thylazetidine-3-carboxylate (1);
ethyl 3-(3,4-difluorophenyl)-1-ethylazet-idine-3-carboxylate (2);
ethyl 3-(3,4-difluorophenyl)-1-methylazet-idine-3-carboxylate (3);
ethyl 3-(3,4-difluorophenyl)azetidine-3-carboxylate (4);
ethyl 3-(3-fluorophenyl)-2,4-dimethylazet-idine-3-carboxylate (5);
ethyl 3-(3-fluorophenyl)-2-methylazetidine-3-carboxylate (6);
ethyl 3-(4-methylphenyl)azetidine-3-carboxylate (7);
methyl 2-methyl-3-(3-methylphenyl)azetidine-3-carboxylate (8);
methyl 3-(3,4-difluorophenyl)-1-ethyl-2-methylazetidine-3-carboxylate (9);
methyl 3-(3,4-difluorophenyl)-2-methylazet-idine-3-carboxylate (10);
ethyl 2-methyl-3-(3-methylphenyl)azetidine-3-carboxylate (11);
ethyl 1-ethyl-3-(4-methylphenyl)azetidine-3-carboxylate (12);
ethyl 3-(3,4-difluorophenyl)-2,4-dimethylazetidine-3-carboxylate (13);
methyl 3-(3,4-difluorophenyl)-1-ethyl-2,4-dimethylazetidine-3-carboxylate (14);
methyl 1-ethyl-2-methyl-3-(3-methylphenyl)azetidine-3-carboxylate (15);
ethyl 3-(3-methylphenyl)azetidine-3-carboxylate (16);
ethyl 3-(3-fluorophenyl)-1,2,4-trimethylazetidine-3-carboxylate (17);
ethyl 1-ethyl-2,4-dimethyl-3-(3-methylphenyl)azetidine-3-carboxylate (18);
ethyl 3-(3,4-difluorophenyl)-2-methylazetidine-3-carboxylate (19);
ethyl 3-(4-methoxyphenyl)azetidine-3-carboxylate (20);
ethyl 1-ethyl-3-(3-methylphenyl)azetidine-3-carboxylate (21);
methyl 1-ethyl-3-(3-methoxyphenyl)-2,4-dimethylazetidine-3-carboxylate (22);
ethyl 1-ethyl-2,4-dimethyl-3-(4-methylphenyl)azetidine-3-carboxylate (23);
ethyl 2,4-dimethyl-3-(3-methylphenyl)azetidine-3-carboxylate (24);
methyl 3-(3,4-difluorophenyl)-2,4-dimethylazetidine-3-carboxylate (25);
ethyl 1,2-dimethyl-3-(3-methylphenyl)azetidine-3-carboxylate (26);
methyl 1-ethyl-2,4-dimethyl-3-(3-methylphenyl)azetidine-3-carboxylate (27);
methyl 1-ethyl-2-methyl-3-(4-methylphenyl)azetidine-3-carboxylate (28);
methyl 1-ethyl-3-(4-methoxyphenyl)-2,4-dimethylazetidine-3-carboxylate (29);
methyl 1-ethyl-3-(4-methoxyphenyl)-2-methylazetidine-3-carboxylate (30);
methyl 2,4-dimethyl-3-(4-methylphenyl)azetidine-3-carboxylate (31);
ethyl 3-(3,4-difluorophenyl)-1-ethyl-2-methylazetidine-3-carboxylate (32);
methyl 2-methyl-3-(4-methylphenyl)azetidine-3-carboxylate (33);
methyl 3-(4-methylphenyl)-1,2-dimethylazetidine-3-carboxylate (34);
ethyl 3-(3-fluorophenyl)azetidine-3-carboxylate (35);
ethyl 1,2-dimethyl-3-(4-methylphenyl)azetidine-3-carboxylate (36); and
methyl 1-ethyl-3-(3-fluorophenyl)-2,4-dimethylazetidine-3-carboxylate (37);
or a stereoisomer or a pharmaceutically acceptable salt thereof.

Additionally, or alternatively, the compound of formula (I) is dextrorotatory.

In one aspect is disclosed a pharmaceutical composition comprising an effective amount of one or more compounds, or a stereoisomer or a pharmaceutically acceptable salt thereof, as defined in the present disclosure, together with one or more pharmaceutically acceptable excipient(s). It is to be understood that the one or more compounds, or a stereoisomer or a pharmaceutically acceptable salt thereof, as defined in the present disclosure refers to compounds of formula (I), or a stereoisomer or a pharmaceutically acceptable salt thereof, as defined in the present disclosure.

Additionally, or alternatively, in embodiments a pharmaceutical composition as defined in the present disclosure, in combination with one or more pharmaceutically acceptable carrier(s). Therefore, in embodiments, pharmaceutical compositions comprise an effective amount of one or more compounds of formula (I), or a stereoisomer or a pharmaceutically acceptable salt thereof, together with one or more pharmaceutically acceptable excipient(s) and/or one or more pharmaceutically acceptable carrier (s), or any combination thereof. Preferably, pharmaceutical compositions comprise one compound of formula (I), or a stereoisomer or a pharmaceutically acceptable salt thereof, preferably a pharmaceutically acceptable salt thereof, in combination with one or more pharmaceutically acceptable excipient(s) and one pharmaceutically acceptable carrier.

Additionally, or alternatively, in embodiments the pharmaceutical composition as defined in the present disclosure, in combination with one or more other active ingredient(s). Therefore, additionally, or alternatively, to pharmaceutically acceptable excipient(s) and/or pharmaceutically acceptable carrier(s), pharmaceutical compositions of the present disclosure may comprise an effective amount of one or more compounds of formula (I), or a stereoisomer or a pharmaceutically acceptable salt thereof, in combination with one or more other active ingredient(s). Therefore, in embodiments, pharmaceutical compositions comprise an effective amount of one or more compounds of formula (I), or a stereoisomer or a pharmaceutically acceptable salt thereof, together with one or more pharmaceutically acceptable excipient (s) and/or one or more pharmaceutically acceptable carrier (s) and/or one or more other active ingredient(s), or any combination thereof.

Additionally, or alternatively, the pharmaceutical compositions consist an effective amount of one or more compounds of formula (I), or a stereoisomer or a pharmaceutically acceptable salt thereof, preferably a pharmaceutically acceptable salt thereof, together with one or more pharmaceutically acceptable excipient(s), preferably 1, 2, or 3 pharmaceutically acceptable excipient(s), more preferably 1 pharmaceutically acceptable excipient.

Additionally, or alternatively, the pharmaceutical compositions consist an effective amount of one or more compounds of formula (I), preferably an effective amount of 1 or 2 compounds of formula (I), more preferably 1 compound of formula (I), or a stereoisomer or a pharmaceutically acceptable salt thereof, preferably a pharmaceutically acceptable salt thereof, together with one or more pharmaceutically acceptable excipient(s), preferably 1, 2, or 3 pharmaceutically acceptable excipient(s), and/or one or more pharmaceutically acceptable carrier(s), preferably 1, 2, or 3 pharmaceutically acceptable carrier(s), more preferably 1 pharmaceutically acceptable carrier.

Additionally, or alternatively, the pharmaceutical compositions consist an effective amount of one or more compounds of formula (I), preferably an effective amount of 1 or 2 compounds of formula (I), more preferably 1 compound of formula (I), or a stereoisomer or a pharmaceutically acceptable salt thereof, preferably a pharmaceutically acceptable salt thereof, together with one or more pharmaceutically acceptable excipient(s), preferably 1, 2, or 3 pharmaceutically acceptable excipient(s), and/or one or more pharmaceutically acceptable carrier(s), preferably 1, 2, or 3 pharmaceutically acceptable carrier(s), more preferably 1 pharmaceutically acceptable carrier, and/or one or more other active ingredient(s), preferably one other active ingredient.

In one aspect is disclosed a compound, or a stereoisomer or pharmaceutically acceptable salt thereof, as defined in the present disclosure, for use as a medicament.

In one aspect is disclosed a compound, or a stereoisomer or pharmaceutically acceptable salt thereof, as defined in the present disclosure, for use in the treatment or prevention of drug addiction or a CNS related disease or condition.

Additionally, or alternatively, in embodiments the drug addiction or the CNS related disease or condition is selected from the group consisting of stimulant addiction, ADHD, ADD, sluggish cognitive tempo, concentration deficit disorder, motivational or reward system dysfunction, autism spectrum disorder, disruptive, impulse control, and conduct disorders, anxiety disorders, eating disorders, depression, dysthymia, Alzheimer's disease, Parkinson's disease, hyperactivity, narcolepsy, and alcoholism.

Additionally, or alternatively, the stimulant is selected from the group consisting of cocaine, amphetamine, and methylphenidate.

Additionally, or alternatively, the attention deficit hyperactivity disorder (ADHD) is selected from the group consisting of attention deficit hyperactivity disorder predominantly inattentive (ADHD-PI or ADHD-I), attention deficit hyperactivity disorder predominantly hyperactive-impulsive (ADHD-PH or ADHD-HI), and attention deficit hyperactivity disorder combined type (ADHD-C).

Additionally, or alternatively, the motivational or reward system dysfunction is selected from the group consisting of anhedonia, apathy, akinetic mutism, abulia, avolition, psychomotor retardation, slowing, and anergia.

Additionally, or alternatively, the disruptive, impulse control, and conduct disorders is selected from the group consisting of oppositional defiant disorder, and obsessive-compulsive disorder.

Additionally, or alternatively, the anxiety disorders are selected from the group consisting of generalized anxiety disorder (GAD), phobias, panic disorder, agoraphobia, social anxiety disorder, post-traumatic stress disorder, separation anxiety disorder, obsessive-compulsive disorder, and selective mutism.

Additionally, or alternatively, the eating disorders are selected from the group consisting of obesity, binge-eating disorder, bulimia nervosa, other specified feeding or eating disorder (OSFED), anorexia nervosa, bulimia nervosa, and binge-purge subtype of anorexia nervosa.

Further, a compound, or a stereoisomer or pharmaceutically acceptable salt thereof, as disclosed herein and herafter may also be beneficial for use in the treatment or prevention of a disease or condition that is associated with HIV-1 reverse transcriptase, oxidosqualene cyclase, aldose reductase, or hepatitis C virus NS5B RNA-dependent RNA polymerase.

Additionally, or alternatively, the disease or condition that is associated with HIV-1 reverse transcriptase, oxidosqualene cyclase, aldose reductase, or hepatitis C virus NS5B RNA-dependent RNA polymerase is selected from the group consisting of acquired immunodeficiency syndrome (AIDS), hypercholesterolemia, Chagas' disease, diabetes mellitus, hepatitis C.

In one aspect is provided use of a compound of formula (I), or a stereoisomer or pharmaceutically acceptable salt thereof, as a nootropic compound.

Substitution, especially fluorination, at the position of metabolic attack - mainly at positions corresponding to substituents R², R³, and R⁴ of compounds having formula (I) may be used to alter the route and rate of metabolic degradation. Furthermore, substitution, especially alkylation and/or halogenation, in particular methyl and/or F, mainly at positions corresponding to substituents R², R³, and/or R⁴ may alter the binding affinity to a protein and/or lipophilicity, conformation, electrostatic potential, dipoles, and pKa of the compound of formula (I). Substitution, in particular fluorination and/or alkylation, may also alter the tissue distribution, pharmacodynamics, and toxicology of the compound. It can be generalized that replacing hydrogen with fluorine at positions corresponding to substituents R², R³, and/or R⁴ may cause minimal steric effects at the receptor.

The compounds as disclosed herein and hereafter may be actively transported over the blood brain barrier by organic cation transporters and may be concentrated in the brain. Compounds as disclosed herein and hereafter may not be oxidized to form epoxides thereby making them less prone to cause oxidative stress. The compounds as disclosed herein and hereafter may be associated with neuroprotective and neuroregenerative properties instead of neurotoxicity or neuro-degeneration.

The compounds as disclosed herein and hereafter may be used, in addition to in treatment or prevention of e.g. Alzheimer's disease and Parkinson's disease, in treatment or prevention of addictions in general. Number of positive pharmacological responses can be achieved simultaneously. While decreasing the tendency to relapse and likelihood of developing an addiction, these compounds may act as general mood stabilizers and general neuroprotectants possessing remarkable antiparkinsonian and antiepileptic character.

Without being limited to any particular theory or mechanism, it is believed that the mechanism of action of the disclosed compounds involve proteins in the mesocorticolimbic dopamine pathway and/or the locus coeruleus-noradrenergic system. The invention is based on the realization that the compounds of the invention bind to and/or affect the activity of proteins that are associated with drug addiction or a CNS related disease or condition.

Also disclosed is a method of treating or preventing a disease or condition selected from the group consisting of drug addiction or a CNS related disease or condition is selected from the group consisting of stimulant addiction, ADHD, ADD, sluggish cognitive tempo, concentration deficit disorder, motivational or reward system dysfunction, autism spectrum disorder, disruptive, impulse control, and conduct disorders, anxiety disorders, eating disorders, depression, dysthymia, Alzheimer's disease, Parkinson's disease, hyperactivity, narcolepsy, and alcoholism in a patient in need thereof, comprising administering a compound as disclosed herein and hereafter, or a stereoisomer or a pharmaceutically acceptable salt thereof, to the patient.

Additionally, or alternatively, the compound, or the stereoisomer or pharmaceutically acceptable salt thereof, as disclosed herein and hereafter for use in treatment or prevention of drug addiction or a CNS related disease or condition requiring the inhibition or activation of at least one protein selected from the group consisting of trace amine receptors (trace amine-associated receptors (TAARs), such as TAAR1), dopamine- and serotonin receptors, dopamine- and serotonin transporters, acyl and methyl transferases, norpinephrine transporter, monoamino-oxidases such as MAO-A, catecholine-O-methylransferase (COMT), adrenergic receptors, tyrosine hydroxylase, histamine receptors such as H1 histamine receptor, orexin receptors, NMDA-receptors, sigma-1 receptor, muscarinic and nicotinic acetylcholine receptors such as M1, M3, and M4 muscarinic acetylcholine receptors, opioid receptors such as µ-opioid receptors (MOR), neuropeptide receptors such as Neuropeptide Y2 receptor, melanocortin receptors (excluding MC3R), neurokinin receptors such as NKR2 and NK3R, corticotropin-releasing factor receptor 1, acetylcholinesterase, 1B melatonin receptor, cholinergic receptors, and dopamine beta-hydroxylase (dopamine beta-monooxygenase, DBH).

In one aspect is disclosed a method for the preparation of a compound of formula (I), or pharmaceutically acceptable salt or a stereoisomer thereof, as defined in the present disclosure, comprising:
(i) providing a compound of formula (II) 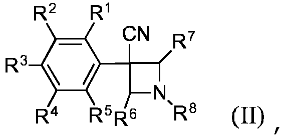 wherein
   R¹, R², R³, R⁴, R⁵, R⁶, and R⁷ are as defined in the present disclosure; and R⁸ is methyl, ethyl, CH₂Ph(*o-*OMe), or a protecting group;
(ii) reacting the compound of formula (II) with one or more compounds each independently selected from the group consisting of acids, bases, and water; or
   reacting the compound of formula (II) with methanol or ethanol, and a Lewis catalyst;
(iii) optionally performing an esterification with a compound of formula (III)

   R¹¹-R" (III),

   wherein
   R¹¹ is methyl or ethyl; and
   R'' is selected from the group consisting of halogen, SR‴, OR‴, and a first activating group, wherein R‴ is H, or a second activating group, optionally in the presence of one or more activating group reactant(s) and/or one or more activating agent(s);
(iv) optionally performing one or more first deprotection reaction(s);
(v) optionally performing a transesterification with a compound of formula (III), wherein R¹¹ is methyl or ethyl; and R'' is selected from the group consisting of SH, and OH, optionally in the presence of one or more activating group reactant(s) and/or one or more activating agent(s);
(vi) optionally performing an amidation with a compound of formula (IV)

   R¹²-R^{8'} (IV),

   wherein
   R¹² is HO, R¹³O, or R¹³, wherein R¹³ is an activating group; and
   R^{8'} is C(O)CH₂NHR¹⁴, C(O)CH(NHR¹⁴)(CH₂)₄NHR¹⁴, C(O)CH(NHR¹⁴)(CH₂)₃N(R¹⁴)C(NR¹⁴)(NHR¹⁴), or 5- (1, 2-dithio-lan-3-yl)valeryl, wherein each R¹⁴ is independently selected from the group consisting of H, and protecting groups, optionally in the presence of one or more activating group reactant(s) and/or one or more activating agent (s) ;
(vii) optionally performing a reductive alkylation with a compound of formula (V)

   R^{8"}-CHO (V),

   wherein R^{8"} is H, or CH₃; and a reducing agent;
(viii) optionally performing an *N*-alkylation with a compound of formula (VI)

   R^{8‴}-R¹⁵ (VI),

   wherein
   R^{8‴} is methyl, ethyl, (*o*-OMe)PhCH₂, or (Ph)CH(CH₂CN); and
   R¹⁵ is selected from the group consisting of halogen, SR¹⁶, and OR¹⁶, wherein R¹⁶ is an activating group, optionally in the presence of one or more activating group reactant(s) and/or one or more activating agent(s);
(ix) optionally performing one or more second deprotection reaction(s);
   to obtain the compound of formula (I), wherein R¹, R², R³, R⁴, R⁵, R⁶, R⁷, R⁸, R⁹ and R¹⁰ are as defined in the present disclosure; and
(x) optionally converting the compound of formula (I) to a pharmaceutically acceptable salt thereof.

Additionally, or alternatively, in (i), R⁸ is methyl, ethyl, CH₂Ph(*o*-OMe), or a protecting group, preferably the protecting group is benzyl.

Additionally, or alternatively, in (ii), reacting the compound of formula (II) with one or more compounds each independently selected from the group consisting of acids, bases, and water, preferably the one or more compounds are HCl and water, preferably the one or more compounds are two compounds, wherein the two compounds are HCl and water. A compound of formula (I), wherein R⁹ is CO₂H; R⁸ is as defined for the compound of formula (II) as defined in the present disclosure; and R¹, R², R³, R⁴, R⁵, R⁶, and R⁷ are as defined in the present disclosure, is formed.

Additionally, or alternatively, in ii), reacting the compound of formula (II) with methanol or ethanol, and a Lewis catalyst, preferably the Lewis catalyst is trimethylsilyl triflate. A compound of formula (I), wherein R⁹ is CO₂R¹⁰, R¹⁰ is methyl or ethyl, R⁸ is as defined for the compound of formula (II) as defined in the present disclosure; and R¹, R², R³, R⁴, R⁵, R⁶, and R⁷ are as defined in the present disclosure, is formed.

Additionally, or alternatively, the method comprises (iii) performing an esterification with a compound of formula (III),

R¹¹-R" (III),

wherein
R¹¹ is methyl or ethyl; and
R" is selected from the group consisting of halogen, SR‴, OR‴, and a first activating group, wherein R‴ is H, or a second activating group, optionally in the presence of one or more activating group reactant(s) and/or one or more activating agent(s), preferably the halogen is Cl, Br, or I; the first activating group is selected from the group consisting of a sulfonate, preferably phenylsulfonate, tosylate, mesylate, or triflate; a halogen, preferably Cl, Br, or I; an amino group, preferably 1-imidazolyl; an alkoxy, preferably *1H*-1,2,3-benzotriazol-1-olate; R‴ is H, or a second activating group, preferably the second activating group is selected from the group consisting of a sulfonyl, preferably tosyl (Ts), mesyl, or trifyl; and an amino group, preferably benzotriazolyl, preferably in the presence of one activating group reactant and/or one or more activating agent(s), wherein the one activating group reactant is 1,1'-carbonyldiimidazole (CDI), diethyl azodicarboxylate (DEAD), diisopropyl azodicarboxylate (DIAD), *N,N*'-dicyclohexylcarbodiimide (DCC), triphenylphosphine (PPh₃), a sulfinic acid, preferably phenylsulfinic acid or p-toluenesulfinic acid; a mesyl halide, preferably methanesulfonyl chloride; trifyl azide, trifluoromethanesulfonyl chloride, a metal halide, preferably NaI; trifluoroacetyl chloride, and trifluoroacetic anhydride; and the one or more activating agent(s) is one activating agent, preferably selected from an acid, preferably HCl, H₂SO₄ or AcOH; a base, preferably NaOH or NaH; water, 4-dimethylaminopyridine (DMAP), triphenylphosphine (PPh₃), a metal halide, preferably NaI; a Lewis acid, preferably TiCl₄, boron trifluoride, and boron trifluoride diethyl etherate. More preferably the compound of formula (III) is methanol or ethanol, in the presence of HCl, or H₂SO₄. A compound of formula (I), wherein R⁹ is CO₂R¹⁰ or COSR¹⁰, preferably CO₂R¹⁰; R¹⁰ is methyl or ethyl; R⁸ is as defined for the compound of formula (II) as defined in the present disclosure; and R¹, R², R³, R⁴, R⁵, R⁶, and R⁷ are as defined in the present disclosure, is formed from a compound of formula (I), wherein R⁹ is CO₂H; R⁸ is as defined for the compound of formula (II) as defined in the present disclosure; and R¹, R², R³, R⁴, R⁵, R⁶, and R⁷ are as defined in the present disclosure.

Additionally, or alternatively, the method comprises (iv) performing one or more first deprotection reaction(s), preferably one first deprotection reaction, more preferably wherein the one or more first deprotection reaction(s) are performed with a reactant or reagent selected from tetra-*n*-butylammonium fluoride, BBr₃, H₂, preferably H₂ in the presence of Pd/C, and 2,3-dichloro-5,6-dicyano-*p*-benzoquinone. One or more protecting group(s) may be removed from a compound of formula (I), wherein R⁹ is CO₂R¹⁰ or COSR¹⁰, preferably CO₂R¹⁰, R¹⁰ is H, provided that R⁹ is CO₂R¹⁰; methyl or ethyl; R⁸ is CH₂Ph(*o*-OMe), or a protecting group; and R¹, R², R³, R⁴, R⁵, R⁶, and R⁷ and are as defined in the present disclosure. It is to be understood that the one or more protecting group(s) that may be removed may be the R⁸ group (i.e., the R⁸ group being a protecting group) or one or more protecting group(s) of the R⁸ group (e.g., one or more of a methyl, TMS, or benzyl), preferably the one or more protecting group(s) is one protecting group, wherein the one protecting group is benzyl or methyl, more preferably the one protecting group is the methyl of CH₂Ph(*o*-OMe) of R⁸ or the one protecting group is R⁸, wherein R⁸ is benzyl, even more preferably the one protecting group is R⁸, wherein R⁸ is benzyl. A compound of formula (I), wherein R⁹ is CO₂R¹⁰ or COSR¹⁰; R¹⁰ is H, provided that R⁹ is CO₂R¹⁰; methyl or ethyl; R⁸ is H, methyl, ethyl, or CH₂Ph(o-OH); and R¹, R², R³, R⁴, R⁵, R⁶, and R⁷ are as defined in the present disclosure, is formed.

Additionally, or alternatively, the method comprises (v) performing a transesterification with a compound of formula (III), wherein R¹¹ is methyl or ethyl; and R'' is selected from the group consisting of SH, and OH, optionally in the presence of one or more activating group reactant(s) and/or one or more activating agent(s), preferably in the presence of one activating agent, more preferably in the presence of one activating agent selected from the group consisting of an acid, preferably HCl, H₂SO₄ or AcOH, even more preferably the compound of formula (III) is methanol or ethanol, in the presence of HCl, or H₂SO₄. A compound of formula (I), wherein R⁹ is CO₂R¹⁰ or COSR¹⁰, preferably R⁹ is CO₂R¹⁰; R¹⁰ is methyl or ethyl; R⁸ is as defined for the compound of formula (II) as defined in the present disclosure; and R¹, R², R³, R⁴, R⁵, R⁶, and R⁷ are as defined in the present disclosure, is formed from a compound of formula (I), wherein R⁹ is CO₂R¹⁰ or COSR¹⁰, preferably R⁹ is CO₂R¹⁰; R¹⁰ is methyl or ethyl; R⁸ is as defined for the compound of formula (II) as defined in the present disclosure; and R¹, R², R³, R⁴, R⁵, R⁶, and R⁷ are as defined in the present disclosure.

Additionally, or alternatively, the method comprises (vi) performing an amidation with a compound of formula (IV)

R¹²-R^{8'} (IV),

wherein
R¹² is HO, R¹³O, or R¹³, wherein R¹³ is an activating group; and R^{8'} is C(O)CH₂NHR¹⁴, C(O)CH(NHR¹⁴)(CH₂)₄NHR¹⁴, C(O)CH(NHR¹⁴)(CH₂)₃N(R¹⁴)C(NR¹⁴)(NHR¹⁴), or 5- (1,2-dithio-lan-3-yl)valeryl, wherein each R¹⁴ is independently selected from the group consisting of H, and protecting groups, optionally in the presence of one or more activating group reactant(s) and/or one or more activating agent(s), preferably the activating group is selected from Cl, Br, and I; a (substituted) amino group, preferably 1-imidazolyl; an alkoxy, preferably *1H*-1,2,3-benzotriazol-1-olate; each R¹⁴ is independently a protecting group each independently selected from Fmoc and *t*-Boc; and in the presence of one activating group reactant and/or one activating agent, more preferably in the presence of one activating group reactant and one activating agent, even more preferably the activating group reactant(s) is selected from 1,1'-carbonyldiimidazole (CDI), diethyl azodicarboxylate (DEAD), diisopropyl azodicarboxylate (DIAD), *N,N*'-dicyclohexylcarbodiimide (DCC); and the activating agent(s) is selected from 1-hydroxy-7-azabenzotriazole (HOAt), hydroxybenzotriazole (HOBt), and 4-dimethylaminopyridine (DMAP). A compound of formula (I), wherein R⁹ is CO₂R¹⁰ or COSR¹⁰, preferably R⁹ is CO₂R¹⁰; R¹⁰ is methyl or ethyl; R⁸ is C(O)CH₂NHR¹⁴, C(O)CH (NHR¹⁴)(CH₂)₄NHR¹⁴, C(O)CH (NHR¹⁴)(CH₂)₃N(R¹⁴)C(NR¹⁴)(NHR¹⁴), or 5-(1,2-dithio-lan-3-yl)valeryl; and R¹, R², R³, R⁴, R⁵, R⁶, and R⁷ are as defined in the present disclosure, is formed from a compound of formula (I), wherein R⁹ is CO₂R¹⁰ or COSR¹⁰, preferably R⁹ is CO₂R¹⁰; R¹⁰ is methyl or ethyl; R⁸ is H; and R¹, R², R³, R⁴, R⁵, R⁶, and R⁷ are as defined in the present disclosure. It is to be understood that in a method for the preparation of a compound of formula (I), wherein R⁹ is CO₂H or COSH, preferably R⁹ is CO₂H; R⁸ is C(O)CH₂NHR¹⁴, C(O)CH(NHR¹⁴)(CH₂)₄NHR¹⁴, C(O)CH(NHR¹⁴)(CH₂)₃N(R¹⁴)C(NR¹⁴)(NHR¹⁴), or 5-(1,2-dithio-lan-3-yl)valeryl; and R¹, R², R³, R⁴, R⁵, R⁶, and R⁷ are as defined in the present disclosure, the ester group of R⁹ (CO₂R¹⁰ or COSR¹⁰, preferably CO₂R¹⁰; R¹⁰ is methyl or ethyl) may be hydrolysed with a method known to those skilled in the art (e.g., using KOH or LiOH).

Additionally, or alternatively, the method comprises (vii) performing a reductive alkylation with a compound of formula (V)

R^{8"}-CHO (V),

wherein
R^{8"} is H, or CH₃; and a reducing agent. Preferably the reducing agent is sodium borohydride. In embodiments the compound of formula (V) is first reacted with a compound of formula (I), wherein R⁹ is CO₂R¹⁰ or COSR¹⁰, preferably R⁹ is CO₂R¹⁰; R¹⁰ is methyl or ethyl; R⁸ is H; and R¹, R², R³, R⁴, R⁵, R⁶, and R⁷ are as defined in the present disclosure, to form an intermediate compound that is reduced with the reducing agent, preferably sodium borohydride. A compound of formula (I), wherein R⁹ is CO₂R¹⁰ or COSR¹⁰, preferably R⁹ is CO₂R¹⁰; R¹⁰ is methyl or ethyl; R⁸ is methyl or ethyl; and R¹, R², R³, R⁴, R⁵, R⁶, and R⁷ are as defined in the present disclosure, is formed from a compound of formula (I), wherein R⁹ is CO₂R¹⁰ or COSR¹⁰, preferably R⁹ is CO₂R¹⁰; R¹⁰ is methyl or ethyl; R⁸ is H; and R¹, R², R³, R⁴, R⁵, R⁶, and R⁷ are as defined in the present disclosure. It is understood that the formed compound may be further hydrolysed with a method known to those skilled in the art (e.g., using KOH or LiOH) to form a compound of formula (I), wherein R⁹ is CO₂H or COSH, preferably R⁹ is CO₂H.

Additionally, or alternatively, the method comprises (viii) performing an N-alkylation with a compound of formula (VI)

R^{8‴}-R¹⁵ (VI),

wherein
R^{8‴} is methyl, ethyl, (*o*-OMe)PhCH₂, or (Ph)CH(CH₂CN); and R¹⁵ is selected from the group consisting of halogen, SR¹⁶, and OR¹⁶, wherein R¹⁶ is an activating group, optionally in the presence of one or more activating group reactant(s) and/or one or more activating agent(s). In embodiments R¹⁵ is Cl, Br, I, SR¹⁶, or OR¹⁶, wherein R¹⁶ is an activating group; preferably the activating group is selected from a sulfonyl, preferably tosyl (Ts), mesyl, or trifyl. In embodiments (viii) comprises one or more activating group reactant(s) and/or one or more activating agent(s), preferably the one or more activating group reactant(s) and/or one or more activating agent(s)is one activating agent selected from a metal halide, preferably NaI. A compound of formula (I), wherein R⁹ is CO₂R¹⁰ or COSR¹⁰, preferably R⁹ is CO₂R¹⁰; R¹⁰ is methyl or ethyl; R⁸ is methyl, ethyl, (*o*-OMe)PhCH₂, or (Ph)CH(CH₂CN); and R¹, R², R³, R⁴, R⁵, R⁶, and R⁷ are as defined in the present disclosure, is formed from a compound of formula (I), wherein R⁹ is CO₂R¹⁰ or COSR¹⁰, preferably R⁹ is CO₂R¹⁰; R¹⁰ is methyl or ethyl; R⁸ is H; and R¹, R², R³, R⁴, R⁵, R⁶, and R⁷ are as defined in the present disclosure. It is understood that the formed compound may be further hydrolysed with a method known to those skilled in the art (e.g., using KOH or LiOH) to form a compound of formula (I), wherein R⁹ is CO₂H or COSH, preferably R⁹ is CO₂H.

Additionally, or alternatively, the method comprises (ix) performing one or more second deprotection reaction(s), preferably one second deprotection reaction, more preferably wherein the one or more second deprotection reaction(s) are performed with a reactant or reagent selected from bases, preferably NaOH or KOH; tetra-n-butylammonium fluoride; acids, preferably trifluoroacetic acid or HCl; BBr₃, H₂, preferably H₂ in the presence of Pd/C, and 2,3-dichloro-5,6-dicyano-p-benzoquinone. One or more protecting group(s) may be removed from a compound of formula (I), wherein R⁹ is CO₂R¹⁰ or COSR¹⁰, preferably CO₂R¹⁰, R¹⁰ is H, provided that R⁹ is CO₂R¹⁰; methyl or ethyl; R⁸ is C(O)CH₂NHR¹⁴, C(O)CH(NHR¹⁴)(CH₂)₄NHR¹⁴,
C(O)CH(NHR¹⁴)(CH₂)₃N(R¹⁴)C(NR¹⁴)(NHR¹⁴), CH₂Ph(*o*-OMe), or a protecting group, wherein each R¹⁴ is independently selected from the group consisting of H, and protecting groups; and R¹, R², R³, R⁴, R⁵, R⁶, and R⁷ and are as defined in the present disclosure to form a compound of formula (I), wherein R⁹ is CO₂R¹⁰ or COSR¹⁰, preferably CO₂R¹⁰; R¹⁰ is H, methyl, or ethyl; R⁸ is H, CH₂Ph(*o*-OH), C(O)CH₂NH₂, C(O)CH(NH₂)(CH₂)₄NH₂, or C(O)CH(NH₂)(CH₂)₃ N (H) C(NH) (NH₂) ; and R¹, R², R³, R⁴, R⁵, R⁶, and R⁷ are as defined in the present disclosure. It is to be understood that any of said protecting group(s) that may be removed may be the R⁸ group, preferably R⁸ is benzyl; or one or more protecting group(s) of the R⁸ group, preferably the methyl of CH₂Ph(*o*-OMe) of R⁸; or one or more protecting groups of R¹⁴ of R⁸, preferably R¹⁴ is t-Boc or Fmoc, or combinations thereof.

Additionally, or alternatively, the method comprises (x) converting the compound of formula (I) to a pharmaceutically acceptable salt thereof.

Additionally, or alternatively, to converting the compound of formula (I) to a pharmaceutically acceptable salt thereof, the method for the preparation of a compound of formula (I), or pharmaceutically acceptable salt or a stereoisomer thereof, further comprises isolating an isomer of the compound of formula (I), preferably isolating an isomer of the compound of formula (I), which is dextrorotatory, wherein said step is performed before or after the step of optionally converting the compound of formula (I) to a pharmaceutically acceptable salt thereof, preferably before the step of optionally converting the compound of formula (I) to a pharmaceutically acceptable salt thereof.

Additionally, or alternatively, to converting the compound of formula (I) to a pharmaceutically acceptable salt thereof, the method for the preparation of a compound of formula (I), or pharmaceutically acceptable salt or a stereoisomer thereof, further comprises converting the compound of formula (I) to another stereoisomer thereof, wherein said step is performed before or after the step of optionally converting the compound of formula (I) to a pharmaceutically acceptable salt thereof.

Additionally, or alternatively, the method further comprises one or more steps of separating and/or purifying one or more compound(s) of formula (I) and/or formula (II).

The compounds as disclosed herein and hereafter can be synthesized using well documented reactions and commercially available starting materials. These novel compounds may be used to achieve desirable pharmacological responses.

Furthermore, compounds of formula (I) may be used as synthesis intermediates for the preparation of other compounds or other pharmaceutically active compositions, which are obtainable from compounds of formula (I) and, for example by introduction of substituents or modification of functional moieties.

The compounds and pharmaceutical compositions as defined in the present disclosure may also be useful in medical devices and medical kits.

### GENERAL PREPARATION METHODS

The compounds according to the present invention may be prepared by processes known per se as follows.

The following non-limiting examples illustrate the preparation of compounds of formula (I). Not all steps or features of the reactions are discussed in detail, as many of the steps or features will be obvious for the person skilled in the art.

### General procedure A for the preparation of compounds of formula (I)

### Step 1:

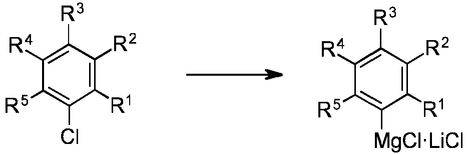

In step 1, a turbo Grignard reagent was prepared as follows (Krasovskiy, A.; Knochel, P. Angew. Chem. Int. Ed. 2004, 43, 3333): a dry and argon flushed flask, equipped with a magnetic stirrer and a septum, was charged with *i*-PrMgCl·LiCl in THF, (1.05-1.2 equiv.) and kept under 15 °C. Aryl halide (such as 4-chloro-1,2-difluorobenzene, 1 equiv.) was dropwise added to the *i-*PrMgCl·LiCl at 0 °C - 15 °C, the formed reaction mixture was stirred at a temperature under 15 °C, and the halogen/Mg exchange was checked by TLC. The formed crude Grignard reagent was used in a subsequent reaction step without purification;

### step 2:

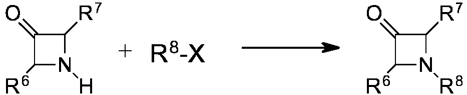

a reaction mixture of optionally substituted 3-azetidinone (1 equiv.), alkylhalide (such as methyl bromide or benzylchloride) (1.05-1.2 equiv.), K₂CO₃ (1.05-1.3 equiv.) and MeOH/isopropylalcohol (10-20 mL/ 10 mmol of 3-azetidinone) was refluxed to form crude optionally substituted 1-alkyl-3-azetidinone (such as 1-benzyl-3-azetidinone or 1-methyl-3-azetidinone) that was used in a subsequent reaction step without purification;

### step 3:

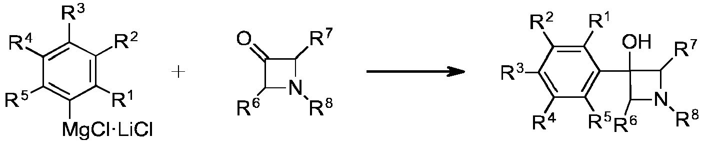

the formed Grignard reagent from step 1 of general procedure A (in THF) was cooled to -10 °C and the formed optionally substituted 1-alkyl-3-azetidinone (1.1-1.2 equiv.) from step 2 of general procedure A was dropwise added. The mixture was stirred for 1-2 h (until completion of the reaction) at an ice bath and was quenched with sat. aqueous NH₄Cl solution. The aqueous phase was extracted with DCM, dried with Na₂SO₄, and concentrated *in vacuo.* The formed crude optionally substituted 1-alkyl-3-phenyl-3-azetidinol was used in a subsequent reaction step without purification;

### step 4:

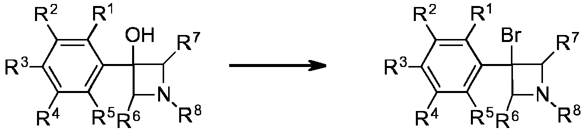

optionally substituted 1-alkyl-3-phenyl-3-azetidinol (1 equiv.) from step 3 of general procedure A was dissolved into ether (20 mL per gram of optionally substituted 1-alkyl-3-phenyl-3-azetidinol) at 0 °C under stirring on an ice bath. PBr₃ (0.41 equiv.) was slowly added to the formed solution to maintain the temperature and stirring was continued further for 2 h. The reaction mixture was poured into basified water, such as saturated NaHCO₃ solution, (6.4 mL per gram of optionally substituted 1-alkyl-3-phenyl-3-azetidinol), the organic phase was separated, and the aqueous phase was extracted twice with diethyl ether (2 ml per gram of optionally substituted 1-alkyl-3-phenyl-3-azetidinol). The ether phases were combined and washed twice with water, dried with MgSO₄, and concentrated in vacuo. The formed crude optionally substituted 3-bromo-1-alkyl-3-phenylazetidine was used in a subsequent reaction step without further purification;

### step 5:

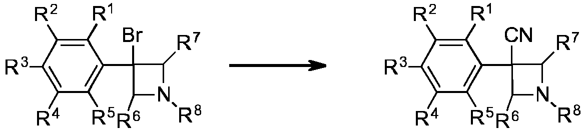

optionally substituted 3-bromo-1-alkyl-3-phenylazetidine (1 equiv.) from step 4 of general procedure A was dissolved into DMF (20 mL / gram) under stirring at 0 °C, then KCN (2.1 equiv.) was added to the solution and the ice-bath was removed. After 1.5 hours the mixture was poured into water (40 mL / gram of optionally substituted 3-bromo-1-alkyl-3-phenylazetidine) and extracted twice with hexane (2 mL / 1 g of the optionally substituted 3-bromo-1-alkyl-3-phenylazetidine). The organic phases were combined, washed twice with water (2 mL / 1g of the optionally substituted 3-bromo-1-alkyl-3-phenylazetidine), dried with MgSO₄ and concentrated in vacuo. The formed crude optionally substituted 1-alkyl-3-phenyl-3-azetidinecarbonitrile was recrystallized from petroleum ether, or used in a subsequent reaction step without further purification;

### step 6 (alternative la):

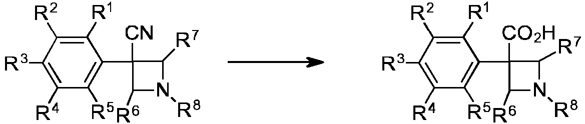

in the case of preparation of a product of formula (I), wherein R⁸ = alkyl (such as methyl, ethyl, or protecting group such as benzyl), and R⁹ = CO₂H, otherwise skip to step 6 (alternative 1b):
a suspension of optionally substituted 1-alkyl-3-phenyl-3-azetidinecarbonitrile from step 5 of general procedure A in an aqueous solution of HCl (pH being 4-5) and toluene was refluxed for 1-2 h followed by an azeotropic distillation, yielding optionally substituted 1-alkyl-3-phenyl-3-azetidinecarboxylic acid that was used in a subsequent reaction step without further purification or optionally purified by flash chromatography; or

### step 6 (alternative 1b):

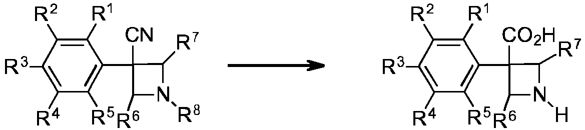

in the case of preparation of a product of formula (I), wherein R⁸ = H, and R⁹ = CO₂H, otherwise skip to Step 6 (alternative 2) :
step 6 (alternative 1a) of general procedure A was followed using 1-alkyl-3-phenyl-3-azetidinecarbonitrile (alkyl = protecting group, such as benzyl) from step 5 of general procedure A, and the crude residue was purified by flash chromatography or used in a subsequent reaction step without purification. The synthesis proceeds with deprotection of optional protecting group (i.e. R⁸ = protecting group, such as benzyl) of the nitrogen according to procedures well known in the art, e.g., with catalytic hydrogenation using H₂ and platinum(IV) oxide (PtO₂), Raney nickel, palladium on carbon (Pd/C) and/or Platinum on carbon (Pt/C). The crude residue was optionally purified by flash chromatography yielding optionally substituted 3-phenyl-3-azetidine-carboxylic acid; or

### step 6 (alternative 2):

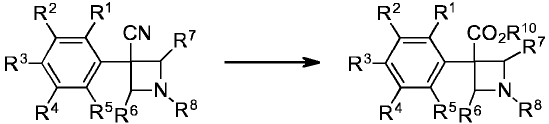

in the case of preparation of a product of formula (I), wherein R⁸ = alkyl (such as methyl, ethyl, or 2-cyano-1-phenylethyl), and R⁹ = CO₂R¹⁰ (R¹⁰ = alkyl such as methyl or ethyl) from the corresponding nitrile, otherwise skip to optional step 7 (alternative 1):
to a suspension of optionally substituted 1-alkyl-3-phenyl-3-azetidinecarbonitrile (3 equiv., from step 5) and methanol or ethanol (1 equiv.), and optionally nitrobenzene (1 equiv.) was added a Lewis catalyst such as trimethylsilyl triflate(TMSOTf, 2 equiv.), and the mixture is stirred at rt for 65 h. H₂O (25 mL) and brine (25 mL) are added, and the mixture is extracted with EtOAc (3 × 30 mL). The combined organic layers are dried (Na₂SO₄) and concentrated. The crude product is purified by flash column chromatography (silica gel) yielding optionally substituted alkyl 1-alkyl-3-phenyl-3-azet-idinecarboxylate (the alkyls may be the same or different alkyls). In the case of protecting group(s) at R⁸, the protecting group(s) was/were removed according to procedures well known in the art, e.g., in the case the protecting group = TIPS, 2.2 mol eq. (TBAF) (1 M solution in THF) was used. The crude product is purified by flash column chromatography (silica gel) yielding the unprotected optionally substituted alkyl 1-alkyl-3-phenyl-3-azetidinecarboxylate (the alkyls may be the same or different alkyls).

### Optional step 7 (alternative 1):

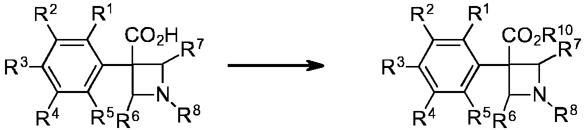

in the case of preparation of a product of formula (I), wherein R⁸ = alkyl (such as methyl, ethyl, or 2-cyano-1-phenylethyl), and R⁹ = CO₂R¹⁰ (R¹⁰ = alkyl such as methyl or ethyl) from a carboxylic acid, otherwise skip to optional step 7 (alternative 2):
a suspension of optionally substituted 1-alkyl-3-phenyl-3-azetidinecarboxylic acid from step 6 (alternative 1a), excess alcohol (methanol or ethanol) (10 mL/ 1 g of the optionally substituted 1-alkyl-3-phenyl-3-azetidinecarboxylic acid), and a catalytic amount of H₂SO₄ was refluxed for 18 h. EtOAc (2 mL/ 1 g of the optionally substituted 1-alkyl-3-phenyl-3-azetidinecarboxylic acid) and a saturated solution of NaHCO₃ (1 mL/ 1 g of the optionally substituted 1-alkyl-3-phenyl-3-azetidinecarboxylic acid) is added, and the mixture is washed with a saturated solution of NaHCO₃ (2 × 1 mL/ 1 g of the optionally substituted 1-alkyl-3-phenyl-3-azetidinecarboxylic acid). The organic layer is dried (Na₂SO₄) and concentrated. The crude product is purified by flash column chromatography (silica gel) yielding optionally substituted alkyl 1-alkyl-3-phenyl-3-azetidinecarboxylate (the alkyls may be the same or different alkyls). In the case of protecting group(s) at R⁸, the protecting group(s) may be removed according to procedures well known in the art, e.g., in the case the protecting group = TIPS, 2.2 mol eq. (TBAF) (1 M solution in THF) was used. The crude product is purified by flash column chromatography (silica gel) yielding the unprotected optionally substituted alkyl 1-alkyl-3-phenyl-3-azetidinecarboxylate (the alkyls may be the same or different alkyls); or

### optional step 7 (alternative 2):

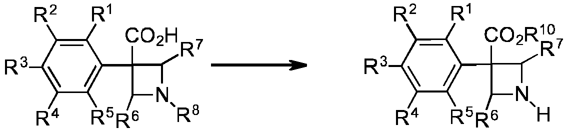

in the case of preparation of a product of formula (I), wherein R⁸ = H, and R⁹ = CO₂R¹⁰ (R¹⁰ = alkyl such as methyl or ethyl) from a protected carboxylic acid (i.e., R⁸ = protecting group), otherwise skip to optional step 7 (alternative 3):
a suspension of optionally substituted 1-alkyl-3-phenyl-3-azetidinecarboxylic acid from step 6 (alternative 1a), excess alcohol (methanol or ethanol) (10 mL/ 1 g of the optionally substituted 1-alkyl-3-phenyl-3-azetidinecarboxylic acid), and a catalytic amount of H₂SO₄ was refluxed for 18 h. EtOAc (2 mL/ 1 g of the optionally substituted 1-alkyl-3-phenyl-3-azetidinecarboxylic acid) and a saturated solution of NaHCO₃ (1 mL/ 1 g of the optionally substituted 1-alkyl-3-phenyl-3-azetidinecarboxylic acid) is added, and the mixture is washed with a saturated solution of NaHCO₃ (2 × 1 mL/ 1 g of the optionally substituted 1-alkyl-3-phenyl-3-azetidinecarboxylic acid). The organic layer is dried (Na₂SO₄) and concentrated. The crude product is purified by flash column chromatography (silica gel) yielding optionally substituted alkyl 1-alkyl-3-phenyl-3-azetidinecarboxylate (the alkyls may be the same or different alkyls). In the case of protecting group(s) present, the protecting group(s) was removed according to procedures well known in the art, e.g., in the case the R⁸ is a protecting group, which is benzyl, with catalytic hydrogenation using H₂ and platinum(IV) oxide (PtO₂), Raney nickel, palladium on carbon (Pd/C) and/or Platinum on carbon (Pt/C). The crude product was optionally purified by flash column chromatography (silica gel) yielding the unprotected optionally substituted alkyl 3-phenyl-3-azetidinecarboxylate; or

### optional step 7 (alternative 3):

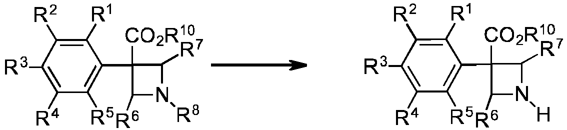

in the case of preparation of a product of formula (I), wherein R⁸ = H, and R⁹ = CO₂R¹⁰ (R¹⁰ = alkyl such as methyl or ethyl) from a protected carboxylic ester (i.e., R⁸ = protecting group), otherwise skip to optional step 7 (alternative 4):
deprotection of the possible protecting group of the nitrogen of the optionally substituted alkyl 1-alkyl-3-phenyl-3-azetidinecarboxylate (i.e., R⁸ = protecting group such as benzyl, e.g. the benzyl of methyl 1-benzyl-3-phenyl-3-azetidinecarboxylate) from step 6 (alternative 2) or optional step 7 (alternative 1) was performed according to procedures well known in the art, e.g., with catalytic hydrogenation using H₂ and platinum(IV) oxide (PtO₂), Raney nickel, palladium on carbon (Pd/C) and/or Platinum on carbon (Pt/C). The crude residue was optionally purified by flash chromatography yielding optionally substituted alkyl 3-phenyl-3-azetidinecarboxylate; or

### optional step 7 (alternative 4):

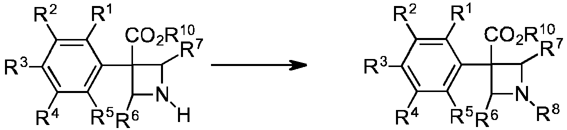

in the case of preparation of a product of formula (I), wherein R⁸ = methyl, ethyl, CH₂Ph(*o*-OMe), or CH₂Ph(*o-*OH), and R⁹ = CO₂R¹⁰ (R¹⁰ = alkyl such as methyl or ethyl) from an optionally substituted alkyl 3-phenyl-3-azetidinecarboxylate, otherwise skip to step 7 (alternative 5) :
a reductive alkylation of the optionally substituted alkyl 3-phenyl-3-azetidinecarboxylate from optional step 7 (alternative 2 or 3) was performed according to procedures well known in the art, e.g., by combining methanal, ethanal, o-[tris(isopropyl)siloxylbenzaldehyde, or 2-methoxybenzaldehyde with the alkyl 3-phenyl-3-azetidinecarboxylate from optional step 7 (alternative 2 or 3), optionally in the presence of one or more water removing compounds or apparatus arranged to remove water, forming the imine. The formed imine was reduced with a reducing agent, such as sodium borohydride. The crude residue was purified by flash chromatography yielding optionally substituted alkyl 1-alkyl-3-phenyl-3-azetidinecarboxylate (the alkyls may be the same or different alkyls). In the case of protecting group(s) at R⁸, the protecting group(s) was/were removed according to procedures well known in the art, e.g., in the case the protecting group = TIPS, 2.2 mol eq. (TBAF) (1 M solution in THF) was used. The crude product was purified by flash column chromatography (silica gel) yielding the unprotected optionally substituted alkyl 1-alkyl-3-phenyl-3-azetidinecarboxylate (the alkyls may be the same or different alkyls); or
an *N*-alkylation of the optionally substituted alkyl 3-phenyl-3-azetidinecarboxylate from optional step 7 (alternative 2 or 3) was performed according to procedures well known in the art, e.g., combining a haloalkane such as methylbromide, ethylbromide, methyliodide, ethyliodide, [o-(bromomethyl)phenoxy]tris(isopropyl)silane, or o-(bromomethyl)methoxybenzene, with the alkyl 3-phenyl-3-azetidinecarboxylate from optional step 7 (alternative 2 or 3), optionally in the presence of one or more activating group reactant(s) and/or one or more activating agent(s) such as sodium iodide. The crude residue was optionally purified by flash chromatography yielding optionally substituted alkyl 1-alkyl-3-phenyl-3-azetidinecarboxylate (the alkyls may be the same or different alkyls). In the case of protecting group(s) at R⁸, the protecting group(s) was/were removed according to procedures well known in the art, e.g., in the case the protecting group = TIPS, 2.2 mol eq. (TBAF) (1 M solution in THF) was used. The crude product is optionally purified by flash column chromatography (silica gel) yielding the unprotected optionally substituted alkyl 1-alkyl-3-phenyl-3-azetidinecarboxylate (the alkyls may be the same or different alkyls); or

### optional step 7 (alternative 5):

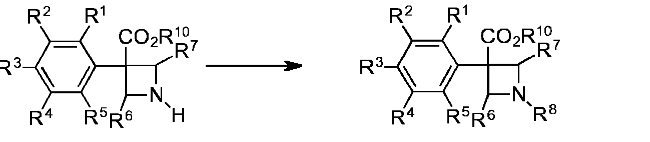

in the case of preparation of a product of formula (I), wherein R⁸ = C(O)CH₂NH₂, C(O)CH(NH₂)(CH₂)₄NH₂, C(O)CH(NH₂)(CH₂)₃NHCNH (NH₂), and R⁹ = CO₂R¹⁰ (R¹⁰ = alkyl such as methyl or ethyl) from a carboxylic ester, otherwise skip to optional step 7 (alternative 6):
an amidation of the optionally substituted alkyl 3-phenyl-3-azetidinecarboxylate from optional step 7 (alternative 2 or 3) was performed according to procedures well known in the art, e.g., combining an optionally protected amino acid (such as *N*-(tert-butoxycarbonyl)glycine) with the optionally substituted alkyl 3-phenyl-3-azetidinecarboxylate from optional step 7 (alternative 2 or 3) with an activating group reactant (such as a carbodiimide such as diethyl azodicarboxylate (DEAD), optionally in the presence of an activating agent (such as 1-hydroxy-7-azabenzotriazole (HOAt). The crude formed residue was purified by flash chromatography or used in an optional subsequent reaction step without purification. In the case of protecting group(s) at R⁸, the protecting group(s) was/were removed according to procedures well known in the art, e.g., in the case of *t*-Boc protected compounds by treating with trifluoroacetic acid in dichloromethane, or with HCl in methanol. The formed crude product was purified by flash chromatography or used in an optional subsequent reaction step without purification; or

### optional step 7 (alternative 6):

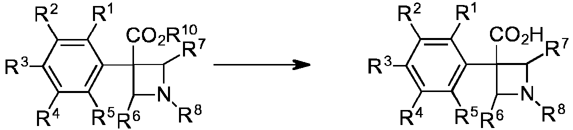

in the case of preparation of a product of formula (I), wherein R⁸ = methyl, ethyl, CH₂Ph(*o*-OMe), CH₂Ph(*o*-OH), C(O)CH₂NH₂, C(O)CH(NH₂)(CH₂)₄NH₂, C(O)CH(NH₂)(CH₂)₃ N(H)C(NH)(NH₂), 5- (1, 2-dithiolan-3-yl) valeryl, and 2-cyano-1-phenylethyl, and R⁹ = CO₂H from a carboxylic ester:
hydrolysis of the carboxylic ester from step 6 (alternative 2), optional step 7 (alternative 1, 4, or 5 was performed according to procedures well known in the art, e.g., reacting the carboxylic ester with a solution comprising NaHCO₃ and water or a solution comprising diluted aqueous NaOH at room temperature. The crude residue was acidified, extracted with EtOAc, dried with MgSO₄, and concentrated in vacuo, and optionally purified by flash chromatography yielding the optionally substituted carboxylic acid.

The pharmaceutically acceptable salts of the compounds of formula (I) may be prepared by conventional processes well-known to the person skilled in the art. For the preparation of pharmaceutical compositions and dosage forms as well as the carriers, diluents and expedients used in the preparation, see, for example, Remington's Pharmaceutical Sciences, 20th Edition, 2000, Mack Publishing Company, Easton, Pennsylvania.

The pharmacological activity of the compounds of the invention may be verified by methods known in the art. For example, the reducing effect on alcohol seeking behavior can be verified using the procedure described by Heidbreder, C.A., et al., Addict Biol. 2007 Mar;12(1):35-50. The Parkinsonism-preventing activity can be shown, for example, as described by Okuda, K., et al. Biol Pharm Bull. 2006 Jul;29(7):1401-1403.

Reference will now be made in detail to some embodiments. The following specific non-limiting examples will further identify the compounds as disclosed herein and hereafter. Not all steps or features of the embodiments are discussed in detail, as many of the steps or features will be obvious for the person skilled in the art based on this specification.

### COMPOUND 1

### Ethyl 3-(3,4-difluorophenyl)-1,2,4-trimethylazetidine-3-carboxylate

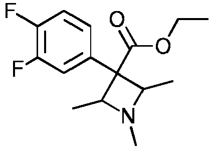

The general procedure A (step 1) was followed starting from 4-chloro-1,2-difluorobenzene. 2,4-Dimethyl-3-azetidinone and methyl iodide were used in step 2 of the general procedure A. Subsequently, steps 3-5, step 6 (alternative 1a), and optional step 7 (alternative 1, wherein ethanol was used), of the general procedure A were followed. ¹H NMR (500 MHz, DMSO-d6) δ 7.34 - 7.16 (m, 3H), 4.20 (q, J = 6.0 Hz, 2H), 3.83 (ddddd, J = 21.6, 7.7, 6.2, 4.7, 1.5 Hz, 2H), 2.41 (d, J = 3.0 Hz, 3H), 1.25 - 1.17 (m, 9H).

### COMPOUND 2

### Ethyl 3-(3,4-difluorophenyl)-1-ethylazetidine-3-carboxylate

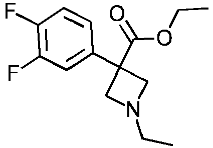

The general procedure A (step 1) was followed starting from 4-chloro-1,2-difluorobenzene. 3-Azetidinone and ethyl iodide were used in step 2 of the general procedure A. Subsequently, steps 3-5, step 6 (alternative 1a), and optional step 7 (alternative 1, wherein ethanol was used), of the general procedure A were followed. ¹H NMR (500 MHz, DMSO-d6) δ 7.29 - 7.15 (m, 3H), 4.24 (q, J = 6.0 Hz, 2H), 3.94 (d, J = 11.5 Hz, 2H), 3.49 (s, 2H), 2.64 - 2.58 (m, 2H), 1.22 (t, J = 6.0 Hz, 3H), 1.07 (t, J = 6.0 Hz, 3H).

### COMPOUND 3

### Ethyl 3-(3,4-difluorophenyl)-1-methylazetidine-3-carboxylate

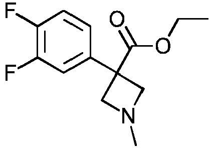

The general procedure A (step 1) was followed starting from 4-chloro-1,2-difluorobenzene. 3-Azetidinone and methyl iodide were used in step 2 of the general procedure A. Subsequently, steps 3-5, step 6 (alternative 1a), and optional step 7 (alternative 1, wherein ethanol was used), of the general procedure A were followed. ¹H NMR (500 MHz, DMSO-d6) δ 7.29 - 7.14 (m, 3H), 4.23 (q, J = 6.1 Hz, 2H), 3.95 (d, J = 11.5 Hz, 2H), 3.53 (dd, J = 11.5, 7.1 Hz, 2H), 2.34 (s, 3H), 1.22 (t, J = 6.0 Hz, 3H).

### COMPOUND 4

### Ethyl 3-(3,4-difluorophenyl)azetidine-3-carboxylate

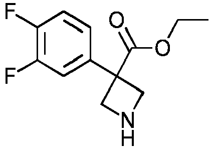

The general procedure A (step 1) was followed starting from p-chlorotoluene. 3-Azetidinone and benzyl bromide were used in step 2 of the general procedure A. Subsequently, steps 3-5, step 6 (alternative 1a), and optional step 7 (alternative 2, wherein ethanol was used, and removal of the benzyl protecting group using H₂ and Pd/C), of the general procedure A were followed. ¹H NMR (500 MHz, DMSO-d6) δ 7.29 - 7.20 (m, 2H), 7.20 - 7.13 (m, 1H), 4.23 (q, J = 6.1 Hz, 2H), 4.00 (dd, J = 13.1, 3.5 Hz, 2H), 3.73 (dd, J = 13.0, 3.5 Hz, 2H), 3.57 (pent, J = 3.5 Hz, 1H), 1.22 (t, J = 6.0 Hz, 3H).

### COMPOUND 5

### Ethyl 3-(3-fluorophenyl)-2,4-dimethylazetidine-3-carboxylate

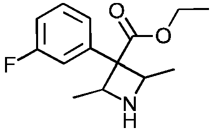

The general procedure A (step 1) was followed starting from m-chlorofluorobenzene. 2,4-Dimethyl-3-azetidinone and benzyl bromide were used in step 2 of the general procedure A. Subsequently, steps 3-5, step 6 (alternative 1a), and optional step 7 (alternative 2, wherein ethanol was used, and removal of the benzyl protecting group using H₂ and Pd/C), of the general procedure A were followed. ¹H NMR (500 MHz, DMSO-d6) δ 7.34 (dddd, J = 7.9, 7.4, 5.0, 0.5 Hz, 1H), 7.30 - 7.19 (m, 2H), 7.10 (dddd, J = 10.1, 7.3, 2.2, 1.3 Hz, 1H), 4.28 - 4.16 (m, 4H), 3.00 (t, J = 6.9 Hz, 1H), 1.22 (t, J = 6.0 Hz, 3H), 1.08 (d, J = 4.7 Hz, 6H).

### COMPOUND 6

### Ethyl 3-(3-fluorophenyl)-2-methylazetidine-3-carboxylate

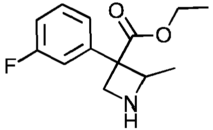

The general procedure A (step 1) was followed starting from m-chlorofluorobenzene. 2-Methyl-3-azetidinone and benzyl bromide were used in step 2 of the general procedure A. Subsequently, steps 3-5, step 6 (alternative 1a), and optional step 7 (alternative 2, wherein ethanol was used, and removal of the benzyl protecting group using H₂ and Pd/C), of the general procedure A were followed. ¹H NMR (500 MHz, DMSO-d6) δ 7.36 - 7.18 (m, 3H), 7.10 (dddd, J = 10.1, 7.3, 2.1, 1.3 Hz, 1H), 4.29 - 4.10 (m, 3H), 3.97 (dd, J = 13.0, 3.6 Hz, 1H), 3.74 (ddd, J = 13.0, 3.6, 1.0 Hz, 1H), 3.24 - 3.18 (m, 1H), 1.22 (t, J = 6.0 Hz, 3H), 1.09 (d, J = 4.8 Hz, 3H).

### Reference COMPOUND 7

### Ethyl 3-(4-methylphenyl)azetidine-3-carboxylate

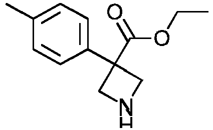

The general procedure A (step 1) was followed starting from p-chloromethylbenzene. 3-Azetidinone and benzyl bromide were used in step 2 of the general procedure A. Subsequently, steps 3-5, step 6 (alternative 1a), and optional step 7 (alternative 2, wherein ethanol was used, and removal of the benzyl protecting group using H₂ and Pd/C), of the general procedure A were followed. ¹H NMR (500 MHz, DMSO-d6) δ 7.33 - 7.27 (m, 2H), 7.10 (dp, J = 8.3, 0.6 Hz, 2H), 4.23 (q, J = 6.0 Hz, 2H), 3.97 (dd, J = 13.0, 3.5 Hz, 2H), 3.66 (dd, J = 13.1, 3.5 Hz, 2H), 3.59 - 3.52 (m, 1H), 2.30 (d, J = 1.3 Hz, 3H), 1.22 (t, J = 6.0 Hz, 3H).

### COMPOUND 8

### Methyl 2-methyl-3-(3-methylphenyl)azetidine-3-carboxylate

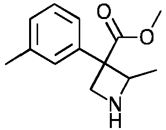

The general procedure A (step 1) was followed starting from m-chlorotoluene. 2-Methyl-3-azetidinone and benzyl bromide were used in step 2 of the general procedure A. Subsequently, steps 3-5, step 6 (alternative 1a), and optional step 7 (alternative 2, wherein methanol was used, and removal of the benzyl protecting group using H₂ and Pd/C) of the general procedure A were followed. ¹H NMR (500 MHz, DMSO-d6) δ 7.30 (ddd, J = 7.7, 2.2, 1.3 Hz, 1H), 7.26 - 7.19 (m, 2H), 7.11 (dddd, J = 7.3, 2.1, 1.3, 0.7 Hz, 1H), 4.18 (dqd, J = 5.8, 4.8, 1.0 Hz, 1H), 3.98 (dd, J = 13.0, 3.6 Hz, 1H), 3.73 (ddd, J = 12.9, 3. 7, 1.0 Hz, 4H), 3.20 (dt, J = 5.9, 3.6 Hz, 1H), 2.31 (d, J = 1.2 Hz, 3H), 1.10 (d, J = 4.8 Hz, 3H).

### COMPOUND 9

### Methyl 3-(3,4-difluorophenyl)-1-ethyl-2-methylazetidine-3-carboxylate

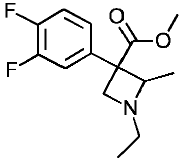

The general procedure A (step 1) was followed starting from 4-chloro-1,2-difluorobenzene. 2-Methyl-3-azetidinone and benzyl bromide were used in step 2 of the general procedure A. Subsequently, steps 3-5, step 6 (alternative 1a), optional step 7 (alternative 2, wherein methanol was used, and removal of the benzyl protecting group), and optional step 7 (alternative 4, reductive alkylation using ethanal and sodium borohydride) of the general procedure A were followed. ¹H NMR (500 MHz, DMSO-d6) δ 7.35 - 7.27 (m, 1H), 7.23 (dddd, J = 10.0, 9.2, 4.7, 0.5 Hz, 1H), 7.19 - 7.12 (m, 1H), 3.73 - 3.65 (m, 2H), 3.60 (s, 3H), 3.53 (dd, J = 11.5, 1.0 Hz, 1H), 2.82 (dq, J = 11.8, 5.9 Hz, 1H), 2.64 (dt, J = 12.0, 5.9 Hz, 1H), 1.20 (d, J = 5.7 Hz, 3H), 1.09 (t, J = 5.9 Hz, 3H).

### COMPOUND 10

### Methyl 3-(3,4-difluorophenyl)-2-methylazetidine-3-carboxylate

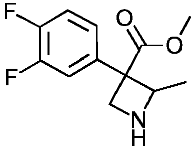

The general procedure A (step 1) was followed starting from 4-chloro-1,2-difluorobenzene. 2-Methyl-3-azetidinone and benzyl bromide were used in step 2 of the general procedure A. Subsequently, steps 3-5, step 6 (alternative 1a), and optional step 7 (alternative 2, wherein methanol was used, and removal of the benzyl protecting group using H₂ and Pd/C) of the general procedure A were followed. ¹H NMR (500 MHz, DMSO-d6) δ 7.30 - 7.19 (m, 2H), 7.16 (dddd, J = 9.2, 3.5, 2.1, 0.3 Hz, 1H), 4.20 (dqd, J = 5.8, 4.8, 1.0 Hz, 1H), 3.95 (dd, J = 13.0, 3.6 Hz, 1H), 3.75 (ddd, J = 13.0, 3.6, 1.0 Hz, 4H), 3.21 (dt, J = 5.9, 3.6 Hz, 1H), 1.10 (d, J = 4.8 Hz, 3H).

### EXPERIMENTS RELATING TO BINDING AFFINITIES OF THE COMPOUNDS OF THE INVENTION

Calculations of binding affinities of the compounds of the invention were performed using Qvina2 using the value 5061982 for explicit random seed. Exhaustiveness was 100. The method of performing binding affinity -calculations of the compounds of the invention is well known in the art and also described in Alhossary A. et al. Fast, Accurate, and Reliable Molecular Docking with QuickVina 2 Bioinformatics (2015) 31 (13) 2214-2216, O. Trott, A. J. Olson, AutoDock Vina: improving the speed and accuracy of docking with a new scoring function, efficient optimization and multithreading, Journal of Computational Chemistry 31 (2010) 455-461, Feinstein WP, Brylinski M. Calculating an optimal box size for ligand docking and virtual screening against experimental and predicted binding pockets. J. Cheminform. (2015), 7 (1) : 18, Tetko, I. V. et al. Virtual computational chemistry laboratory - design and description, J. Comput. Aid. Mol. Des., 2005, 19, 453-63, VCCLAB, Virtual Computational Chemistry Laboratory, http://www.vcclab.org, 2005. The search space (X,Y,Z)-coordinates of the centre for the calculations of binding affinities can be obtained with methods well known in the art. E.g., the search space (X,Y,Z)-coordinates for PDB ID 1bp3 and protein homolog ClassA_5ht1a_human_Active_6G79_2018-07-10_GPCRDB were (13.730, 30.880, 13.170) and (91.84457623, 54.99481, 63.31365), respectively. The PDB IDs and the protein homolog codes of proteins used in the calculations of the compounds of the invention are listed in Table 1 and some of the results of the calculations are presented in Table 2.

**Table 1. The PDB IDs and the protein homolog codes of proteins used in the calculations of binding affinities of compounds disclosed herein and hereafter.**

| Entry | PDB ID¹ | Entry | Homolog² |
|---|---|---|---|
| 1 | 1bp3 | 42 | ClassA_5ht1a_human_Active_6G79_2018-07-10_GPCRDB |
| 2 | 1h22 | 43 | ClassA_5ht2a_human_Active_6BQG_2018-07-10_GPCRDB |
| 3 | 1kux | 44 | ClassA_ada1a_human_Active_3SN6_2018-07-10_GPCRDB |
| 4 | 1n7i | 45 | ClassA_ada1b_human_Active_3SN6_2018-07-10_GPCRDB |
| 5 | 2toh | 46 | ClassA_ada2a_human_Active_6G79_2018-07-10_GPCRDB |
| 6 | 2vz2 | 47 | ClassA_ada2b_human_Active_6G79_2018-07-10_GPCRDB |
| 7 | 2xsn | 48 | ClassA_ada2c_human_Active_6G79_2018-07-10_GPCRDB |
| 8 | 2z5x | 49 | ClassA_drd1_human_Active_3SN6_2018-07-10_GPCRDB |
| 9 | 3bwm | 50 | ClassA_hrh3_human_Active_4MQT_2018-07-10_GPCRDB |
| 10 | 3hcd | 51 | ClassA_mtr1a_human_Active_3SN6_2018-07-10_GPCRDB |
| 11 | 3pbl | 52 | ClassA_mtr1b_human_Active_5TUD_2018-07-10_GPCRDB |
| 12 | 3qel | 53 | ClassA_taar1_human_Active_3SN6_2018-07-10_GPCRDB |
| 13 | 3rze | 54 | NET_SLC6A2 |
| 14 | 3uon | 55 | neuropeptide_y2_receptor_2ik3 |
| 15 | 3zpq | 56 | neuropeptide_y5_receptor_2he6 |
| 16 | 4a6e | 57 | ClassA_acthr_human_Active_5XRA_2018-07-10_GPCRDB |
| 17 | 4daj | 58 | ClassA_mc3r_human_Active_5XRA_2018-07-10_GPCRDB |
| 18 | 4djh | 59 | ClassA_mc4r_human_Active_5XRA_2018-07-10_GPCRDB |
| 19 | 4k5y | 60 | ClassA_mc5r_human_Active_5XRA_2018-07-10_GPCRDB |
| 20 | 4kfq | 61 | ClassA_mshr_human_Active_5XRA_2018-07-10_GPCRDB |
| 21 | 4ldo | 62 | ClassA_nk1r_human_Active_6B73_2018-07-10_GPCRDB |
| 22 | 4n6h | 63 | ClassA_nk2r_human_Active_6B73_2018-07-10_GPCRDB |
| 23 | 4rvm | 64 | ClassA_nk3r_human_Active_6B73_2018-07-10_GPCRDB |
| 24 | 4s0v | 65 | ClassA_oxyr_human_Active_5DYS_2018-07-10_GPCRDB |
| 25 | 4xp1 | | |
| 26 | 4zj8 | | |
| 27 | 5ain | | |
| 28 | 5c1m | | |
| 29 | 5cxv | | |
| 30 | 5dhh | | |
| 31 | 5dsg | | |
| 32 | 5hk1 | | |
| 33 | 5i73 | | |
| 34 | 5kxi | | |
| 35 | 5pah | | |
| 36 | 5tvn | | |
| 37 | 5v54 | | |
| 38 | 5wiu | | |
| 39 | 5zbq | | |
| 40 | 6bqg | | |
| 41 | 6cm4 | | |

| | | | |
|---|---|---|---|
| ¹ The PDB structures were obtained from the Protein Data Bank: http://www.rcsb.org/. ² Protein homologs were obtained from GPCRdb (http://docs.gpcrdb.org/index.html , Pándy-Szekeres G, Munk C, Tsonkov TM, Mordalski S, Harpsøe K, Hauser AS, Bojarski AJ, Gloriam DE. GPCRdb in 2018: adding GPCR structure models and ligands. 2017, Nucleic Acids Res., Nov 16. http://dx.doi.org/10.1093/nar/gkx1109). | | | |

**Table 2. Results of binding affinity calculations of compounds of the invention performed on proteins, logP and logS values.**

| | | | | Docked protein¹ | | | | | |
|---|---|---|---|---|---|---|---|---|---|
| | | | Search space (x.x.x) in Å | 1 | 2 | 3 | 4 | 5 | 6 |
| Compound | log P | logS | Value* Value *Value | ΔG (kcal/mol) | | | | | |
| 1 | 3.55 | -3.05 | 13.606 | -3.8 | -7.8 | -5.9 | -1.2 | -6.8 | -2.7 |
| 2 | 2.75 | -2.72 | 13.151 | -4.0 | -7.8 | -6.1 | -4.2 | -7.3 | -5.4 |
| 3 | 2.18 | -2.49 | 13.441 | -4.1 | -7.9 | -5.8 | -4.8 | -7.1 | -6.2 |
| 4 | 1.57 | -2.95 | 12.255 | -4.1 | -8.0 | -5.7 | -7.1 | -7.2 | -6.7 |
| 5 | 2.72 | -3.23 | 12.693 | -3.6 | -8.5 | -5.7 | -6.6 | -7.6 | -4.6 |
| 6 | 2.13 | -2.72 | 12.655 | -3.9 | -8.9 | -5.8 | -7.1 | -7.3 | -5.2 |
| 7 | 1.83 | -2.56 | 12.878 | -4.0 | -7.8 | -5.8 | -7.2 | -7.3 | -6.7 |
| 8 | 1.78 | -2.61 | 11.919 | -3.8 | -8.9 | -5.6 | -6.9 | -7.5 | -6.0 |
| 9 | 2.87 | -2.72 | 13.543 | -4.1 | -8.5 | -6.0 | -3.6 | -7.2 | -2.3 |
| 10 | 1.99 | -3.00 | 12.441 | -4.2 | -8.6 | -5.9 | -6.9 | -7.6 | -6.1 |

| | Docked protein¹ | | | | | | | | |
|---|---|---|---|---|---|---|---|---|---|
| | 7 | 8 | 9 | 10 | 11 | 12 | 13 | 14 | 15 |
| Compound | ΔG (kcal/mol) | | | | | | | | |
| 1 | 2.1 | -3.8 | -5.4 | -0.1 | -6.1 | -3.2 | -7.2 | -8.0 | -7.8 |
| 2 | 0.7 | -5.9 | -5.3 | -4.1 | -6.9 | -4.7 | -8.7 | -8.4 | -6.8 |
| 3 | -1.1 | -6.8 | -5.6 | -5.2 | -6.9 | -5.5 | -9.0 | -8.2 | -7.8 |
| 4 | 0.2 | -7.5 | -5.5 | -7.5 | -7.1 | -5.8 | -8.3 | -7.8 | -7.5 |
| 5 | 2.9 | -6.2 | -5.3 | -8.2 | -6.5 | -4.2 | -7.8 | -8.2 | -7.2 |
| 6 | 4.1 | -7.1 | -5.2 | -7.6 | -7.1 | -4.8 | -8.0 | -7.9 | -6.7 |
| 7 | -1.1 | -7.7 | -5.7 | -6.3 | -7.2 | -5.8 | -8.5 | -8.0 | -7.6 |
| 8 | 6.3 | -6.4 | -5.1 | -6.9 | -6.9 | -3.6 | -6.7 | -7.6 | -6.7 |
| 9 | -1.4 | -6.3 | -5.4 | -4.7 | -6.8 | -5.1 | -8.1 | -8.6 | -6.9 |
| 10 | 1.5 | -7.5 | -5.2 | -6.9 | -7.0 | -6.3 | -8.3 | -8.1 | -6.6 |

| | Docked protein¹ | | | | | | | | |
|---|---|---|---|---|---|---|---|---|---|
| | 16 | 17 | 18 | 19 | 20 | 21 | 22 | 23 | 24 |
| Compound | ΔG (kcal/mol) | | | | | | | | |
| 1 | -6.4 | -8.3 | -5.8 | -6.9 | -3.7 | -4.2 | -7.1 | -6.1 | -8.0 |
| 2 | -6.9 | -8.0 | -6.3 | -7.1 | -5.4 | -7.2 | -7.3 | -6.8 | -7.5 |
| 3 | -7.2 | -7.9 | -6.5 | -7.2 | -5.9 | -7.4 | -7.3 | -6.8 | -7.5 |
| 4 | -7.2 | -7.7 | -7.5 | -6.9 | -6.7 | -7.2 | -7.0 | -7.2 | -6.8 |
| 5 | -7.3 | -8.4 | -6.4 | -6.8 | -5.2 | -5.5 | -6.8 | -6.7 | -7.0 |
| 6 | -7.1 | -7.9 | -7.4 | -7.2 | -6.9 | -6.0 | -7.2 | -6.4 | -6.5 |
| 7 | -7.0 | -7.6 | -7.4 | -7.0 | -7.2 | -7.4 | -6.7 | -7.3 | -6.9 |
| 8 | -7.2 | -7.6 | -7.1 | -7.1 | -6.7 | -6.7 | -6.8 | -6.7 | -6.5 |
| 9 | -7.9 | -8.4 | -6.4 | -7.1 | -4.5 | -7.0 | -7.1 | -6.9 | -7.0 |
| 10 | -7.5 | -8.0 | -7.8 | -7.0 | -7.4 | -5.9 | -7.0 | -7.0 | -6.8 |

| | Docked protein¹ | | | | | | | | |
|---|---|---|---|---|---|---|---|---|---|
| | 25 | 26 | 27 | 28 | 29 | 30 | 31 | 32 | 33 |
| Compound | ΔG (kcal/mol) | | | | | | | | |
| 1 | -7.3 | -7.6 | -4.5 | -7.4 | -7.8 | -5.8 | -8.4 | -6.9 | -7.9 |
| 2 | -8.0 | -6.8 | -4.6 | -7.1 | -8.1 | -5.5 | -8.4 | -7.4 | -7.2 |
| 3 | -8.0 | -6.9 | -5.7 | -6.9 | -8.0 | -5.4 | -8.1 | -7.5 | -7.3 |
| 4 | -7.6 | -6.7 | -6.5 | -6.7 | -7.9 | -5.7 | -8.3 | -7.7 | -7.0 |
| 5 | -7.6 | -7.2 | -6.6 | -7.2 | -8.1 | -5.7 | -8.0 | -7.2 | -7.2 |
| 6 | -7.4 | -6.7 | -6.2 | -7.0 | -7.9 | -5.9 | -8.0 | -7.4 | -7.0 |
| 7 | -7.6 | -6.9 | -6.5 | -6.5 | -7.8 | -5.7 | -7.9 | -7.5 | -7.0 |
| 8 | -7.2 | -7.0 | -6.8 | -6.7 | -8.1 | -5.9 | -8.0 | -7.3 | -6.9 |
| 9 | -7.6 | -7.1 | -5.8 | -7.1 | -8.8 | -6.1 | -8.8 | -8.0 | -7.7 |
| 10 | -7.7 | -7.4 | -6.9 | -6.9 | -8.1 | -5.9 | -8.3 | -7.8 | -7.1 |

| | Docked protein¹ | | | | | | | | |
|---|---|---|---|---|---|---|---|---|---|
| | 34 | 35 | 36 | 37 | 38 | 39 | 40 | 41 | 42 |
| Compound | ΔG (kcal/mol) | | | | | | | | |
| 1 | -1.0 | -6.9 | -7.4 | -8.0 | -7.4 | -7.0 | -6.2 | -6.5 | -7.2 |
| 2 | -3.6 | -6.9 | -6.6 | -7.5 | -6.9 | -7.2 | -7.2 | -6.7 | -6.9 |
| 3 | -4.3 | -6.8 | -7.0 | -7.7 | -7.2 | -6.7 | -7.1 | -6.6 | -7.1 |
| 4 | -4.7 | -6.7 | -6.7 | -7.6 | -6.7 | -6.7 | -7.3 | -7.0 | -7.2 |
| 5 | -2.4 | -7.6 | -6.8 | -7.2 | -6.7 | -6.8 | -7.3 | -6.4 | -6.8 |
| 6 | -3.6 | -7.3 | -6.6 | -7.2 | -7.0 | -6.6 | -7.1 | -7.0 | -6.9 |
| 7 | -4.8 | -6.8 | -6.4 | -7.6 | -7.0 | -6.9 | -6.7 | -7.2 | -6.8 |
| 8 | -3.6 | -7.3 | -6.4 | -7.3 | -7.7 | -6.3 | -7.0 | -6.8 | -7.2 |
| 9 | -2.4 | -7.4 | -7.0 | -8.0 | -6.9 | -7.2 | -7.2 | -7.0 | -7.2 |
| 10 | -4.1 | -6.8 | -6.4 | -7.2 | -7.4 | -6.6 | -7.0 | -7.1 | -7.1 |

| | Docked protein¹ | | | | | | | | |
|---|---|---|---|---|---|---|---|---|---|
| | 43 | 44 | 45 | 46 | 47 | 48 | 49 | 50 | 51 |
| Compound | ΔG (kcal/mol) | | | | | | | | |
| 1 | -7.6 | -6.6 | -7.3 | -5.1 | -6.6 | -2.2 | -7.7 | -5.6 | -5.2 |
| 2 | -7.2 | -6.3 | -6.9 | -6.0 | -6.2 | -3.0 | -7.4 | -5.8 | -6.6 |
| 3 | -7.2 | -6.5 | -7.2 | -5.3 | -6.5 | -3.9 | -7.1 | -5.9 | -6.6 |
| 4 | -7.3 | -6.2 | -7.3 | -4.9 | -6.3 | -3.0 | -7.3 | -5.8 | -5.9 |
| 5 | -6.4 | -6.8 | -7.5 | -4.8 | -7.1 | -3.9 | -7.5 | -6.4 | -5.1 |
| 6 | -7.1 | -6.7 | -7.5 | -5.9 | -6.6 | -4.2 | -7.5 | -6.3 | -6.3 |
| 7 | -6.7 | -6.5 | -7.2 | -5.9 | -6.2 | -4.4 | -7.1 | -5.9 | -6.2 |
| 8 | -6.9 | -6.7 | -7.7 | -4.5 | -6.5 | -2.2 | -7.3 | -5.6 | -5.2 |
| 9 | -7.0 | -7.1 | -6.8 | -5.9 | -6.5 | -3.8 | -8.0 | -6.3 | -6.8 |
| 10 | -7.1 | -6.6 | -7.7 | -6.5 | -6.4 | -3.8 | -7.1 | -6.1 | -5.5 |

| | Docked protein¹ | | | | | | | | |
|---|---|---|---|---|---|---|---|---|---|
| | 52 | 53 | 54 | 55 | 56 | 57 | 58 | 59 | 60 |
| Compound | ΔG (kcal/mol) | | | | | | | | |
| 1 | -8.3 | -8.3 | -8.0 | -6.1 | -6.4 | -6.2 | 0.0 | -7.2 | -6.7 |
| 2 | -7.9 | -7.2 | -7.4 | -7.2 | -6.1 | -5.7 | 0.0 | -7.3 | -6.8 |
| 3 | -8.1 | -7.4 | -7.4 | -7.9 | -6.2 | -5.6 | 0.0 | -7.4 | -7.0 |
| 4 | -7.8 | -7.5 | -7.3 | -8.2 | -6.1 | -5.4 | 0.0 | -7.3 | -7.4 |
| 5 | -8.1 | -8.0 | -7.4 | -7.3 | -6.3 | -5.6 | 0.0 | -7.4 | -7.8 |
| 6 | -7.8 | -7.9 | -7.2 | -7.8 | -6.3 | -5.8 | 0.0 | -7.0 | -7.9 |
| 7 | -7.6 | -7.2 | -7.0 | -7.8 | -6.3 | -5.3 | 0.0 | -7.2 | -7.0 |
| 8 | -7.9 | -7.2 | -7.5 | -7.7 | -6.5 | -6.3 | 0.0 | -7.2 | -7.8 |
| 9 | -8.3 | -7.6 | -7.5 | -7.9 | -6.4 | -6.0 | 0.0 | -6.6 | -7.0 |
| 10 | -7.9 | -7.7 | -7.4 | -7.7 | -6.4 | -5.5 | 0.0 | -7.1 | -7.2 |

| | | Docked protein¹ | | | | | | | |
|---|---|---|---|---|---|---|---|---|---|
| | | 61 | | 62 | | 63 | | 64 | 65 |
| Compound | | ΔG (kcal/mol) | | | | | | | |
| 1 | | -6.2 | | -7.3 | | -7.9 | | -8.4 | -3.7 |
| 2 | | -6.0 | | -6.5 | | -7.5 | | -7.7 | -4.8 |
| 3 | | -5.8 | | -6.3 | | -7.7 | | -7.7 | -5.2 |
| 4 | | -6.4 | | -6.2 | | -7.8 | | -7.5 | -4.9 |
| 5 | | -5.9 | | -6.4 | | -8.1 | | -7.9 | -5.6 |
| 6 | | -6.2 | | -6.2 | | -7.9 | | -7.7 | -4.9 |
| 7 | | -6.3 | | -5.8 | | -7.1 | | -7.2 | -5.3 |
| 8 | | -6.0 | | -6.1 | | -7.5 | | -7.7 | -4.3 |
| 9 | | -6.7 | | -6.7 | | -7.8 | | -8.0 | -5.1 |
| 10 | | -6.0 | | -6.3 | | -7.4 | | -7.9 | -4.7 |

| | | | | | | | | | |
|---|---|---|---|---|---|---|---|---|---|
| ¹ The number of the docked protein corresponds to the entry number found in Table 1. | | | | | | | | | |

The compound with strongest binding affinity for trace amine receptor 1 (TAAR1), norepinephrine transporter (NET), 5-HT2A receptor, 5-HT2B receptor, OX1 orexin receptor, Mu-opioid receptor, Neurokinin 1 receptor (NK1R), and Melanocortin 3 receptor (MC3R) is ethyl 3-(3,4-difluorophenyl)-1,2,4-trimethylazetidine-3-carboxylate (1).

The compound with strongest binding affinity for dopamine transporter (DAT) is ethyl 3-(3,4-difluorophenyl)-1-methylazetidine-3-carboxylate (3). The compound with strongest binding affinity for dopamine 1 (D1) receptor, Sigma-1 receptor, and 1B Melatonin receptor is methyl 1-ethyl-2-methyl-3-(3-methylphenyl)azetidine-3-carboxylate (15):

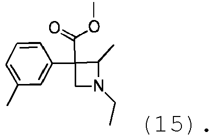

The compound with strongest binding affinity for dopamine 2 (D2) receptor, MAO-A, Alpha-2C adrenergic receptor, α4β2 nicotinic receptor, and CHK1 kinase is ethyl 3-(4-methylphenyl)azetidine-3-carboxylate (7). The compound with strongest binding affinity for dopamine 3 (D3) receptor, and Kappa-opioid receptor is ethyl 3-(3,4-difluorophenyl)-2-methylazetidine-3-carboxylate (19):

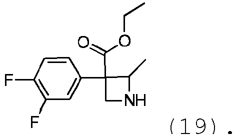

The compound with strongest binding affinity for dopamine 4 (D4) receptor is methyl 2-methyl-3-(3-methylphenyl)azetidine-3-carboxylate (8).

The compound with strongest binding affinity for phenylethanolamine N-methyltransferase (PNMT) is ethyl 3-(3-fluorophenyl)-2,4-dimethylazetidine-3-carboxylate (5).

The compound with strongest binding affinity for serotonin transporter (SERT) is ethyl 3-(4-methoxyphenyl)azetidine-3-carboxylate (20) not part of the invention :

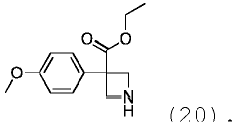

The compound with strongest binding affinity for serotonin N-acetyltransferase is ethyl 1-ethyl-3-(3-methylphenyl)azetidine-3-carboxylate (21):

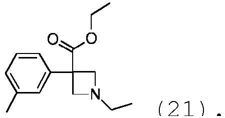

The compound with strongest binding affinity for N-acetylserotonin methyltransferase, 1A Melatonin receptor, and Melanocortin 1 receptor (MSHR, MC1R) is methyl 3-(3,4-difluorophenyl)-1-ethyl-2-methylazetidine-3-carboxylate (9).

The compound with strongest binding affinity for 5-HT1A receptor is methyl 1-ethyl-3-(3-methoxyphenyl)-2,4-dimethylazetidine-3-carboxylate (22):

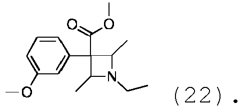

The compound with strongest binding affinity for 5-HT1B receptor, and β1 adrenergic receptor is ethyl 1-ethyl-2,4-dimethyl-3-(4-methylphenyl)azetidine-3-carboxylate (23) :

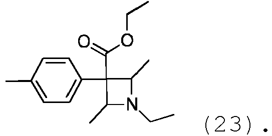

The compound with strongest binding affinity for 5-HT2C receptor, Alpha-1B adrenergic receptor, Alpha-2B adrenergic receptor, Melanocortin 2 receptor (ACTHR, MC2R), and Melanocortin 4 receptor (MC4R) is ethyl 2,4-dimethyl-3-(3-methylphenyl)azetidine-3-carboxylate (24) :

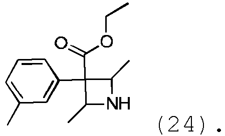

The compound with strongest binding affinity for 5-HT3 receptor is methyl 3-(3,4-difluorophenyl)-2,4-dimethylazetidine-3-carboxylate (25):

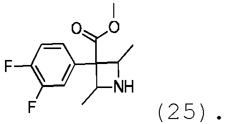

The compound with strongest binding affinity for MAO-B is ethyl 3-(3,4-difluorophenyl)azetidine-3-carboxylate (4) .

The compound with strongest binding affinity for COMT is ethyl 1,2-dimethyl-3-(3-methylphenyl)azetidine-3-carboxylate (26):

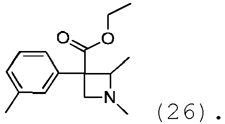

The compound with strongest binding affinity for Alpha-1A adrenergic receptor, and Nociceptin / orphanin receptor is methyl 1-ethyl-2,4-dimethyl-3-(3-methylphenyl)azetidine-3-carboxylate (27):

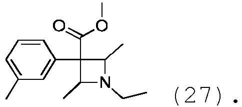

The compound with strongest binding affinity for Alpha-2A adrenergic receptor, and Prolactin receptor is methyl 3-(3,4-difluorophenyl)-2-methylazetidine-3-carboxylate (10).

The compound with strongest binding affinity for β2 adrenergic receptor methyl 1-ethyl-2-methyl-3-(4-methylphenyl)azetidine-3-carboxylate (28):

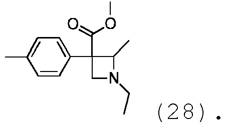

The compound with strongest binding affinity for Phenylalanine hydroxylase is methyl 1-ethyl-3-(4-methoxyphenyl)-2,4-dimethylazetidine-3-carboxylate (29):

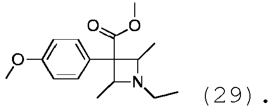

The compounds with strongest binding affinity for Tyrosine hydroxylase are methyl 1-ethyl-3-(4-methoxyphenyl)-2-methylazetidine-3-carboxylate (30): 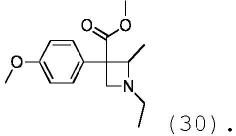 and methyl 2,4-dimethyl-3-(4-methylphenyl)azetidine-3-carboxylate (31):

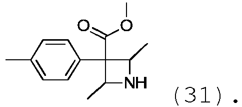

The compound with strongest binding affinity for H1 histamine receptor is ethyl 1-ethyl-3-(4-methylphenyl)azetidine-3-carboxylate (12):

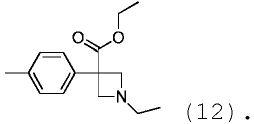

The compound with strongest binding affinity for H3 histamine receptor, and Neurokinin 2 receptor (NK2R) is ethyl 3-(3-fluorophenyl)-1,2,4-trimethylazetidine-3-carboxylate (17):

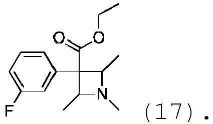

The compound with strongest binding affinity for OX2 orexin receptor is ethyl 3-(3,4-difluorophenyl)-1-ethyl-2-methylazetidine-3-carboxylate (32):

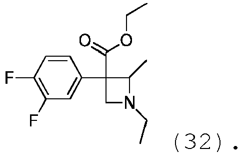

The compound with strongest binding affinity for NMDA receptor GluN1 is methyl 2-methyl-3-(4-methylphenyl)azetidine-3-carboxylate (33):

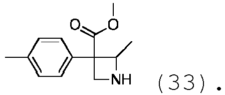

The compound with strongest binding affinity for NMDA receptor Glu2B, M2 muscarinic acetylcholine receptor, and neuropeptide Y1 receptor is ethyl 3-(3,4-difluorophenyl)-2,4-dimethylazetidine-3-carboxylate (13) :

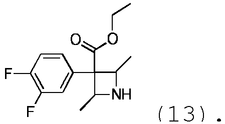

The compound with strongest binding affinity for Delta-opioid receptor, M1 muscarinic acetylcholine receptor, M3 Muscarinic acetylcholine receptor, and M4 muscarinic acetylcholine receptor is methyl 3-(3,4-difluorophenyl)-1-ethyl-2,4-dimethylazetidine-3-carboxylate (14):

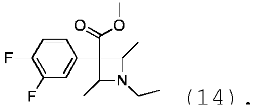

The compound with strongest binding affinity for Acetylcholinesterase, and Melanocortin 5 receptor (MC5R) is ethyl 2-methyl-3-(3-methylphenyl)azetidine-3-carboxylate (11):

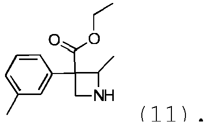

The compound with strongest binding affinity for Neuropeptide Y2 receptor is ethyl 3-(3-methylphenyl) azetidine-3-carboxylate (16) not part of the invention:

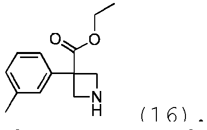

The compound with strongest binding affinity for Neuropeptide Y5 receptor is methyl 3-(4-methylphenyl)-1,2-dimethylazetidine-3-carboxylate (34):

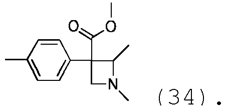

The compound with strongest binding affinity for Neurokinin 3 receptor (NK3R) is ethyl 1-ethyl-2,4-dimethyl-3-(3-methylphenyl)azetidine-3-carboxylate (18) :

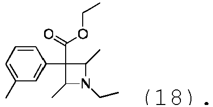

The compound with strongest binding affinity for Oxytocin receptor (OXYR) is ethyl 3-(3-fluorophenyl)azetidine-3-carboxylate (35) not part of the invention:

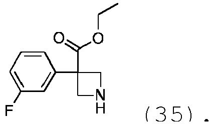

The compound with strongest binding affinity for Corticotropin-releasing factor receptor 1 (CRF1R) is ethyl 1,2-dimethyl-3-(4-methylphenyl)azetidine-3-carboxylate (36):

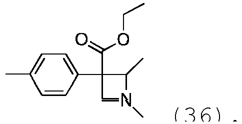

It will be obvious to a person skilled in the art that, as the technology advances, the inventive concept can be implemented in various ways. The invention and its embodiments are not limited to the examples described above but may vary within the scope of the claims.

The embodiments described hereinbefore may be used in any combination with each other. Several of the embodiments may be combined together to form a further embodiment. A product, a system, a method, or a use, disclosed herein, may comprise at least one of the embodiments described hereinbefore. It will be understood that the benefits and advantages described above may relate to one embodiment or may relate to several embodiments.

## Claims

1. A compound of formula (I) 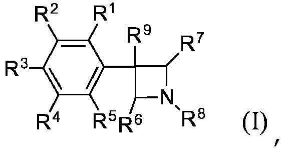 wherein
R¹, R² and R⁵ are H;
R³ and R⁴ are each independently selected from the group consisting of H, halogen, C₁₋₄-alkyl, C₁₋₃-(per) haloalkyl, C₁₋₃-alkoxy, C₁₋₃- (per) haloalkoxy, and OH, with the provision that if one of R³ or R⁴ is H then at least one of R⁶, and R⁷ is each independently selected from the group consisting of methyl, and ethyl, and the other one of R⁶, and R⁷ is selected from the group consisting of H, methyl, and ethyl, or R⁶, and R⁷ are both H, and R⁸ is selected from the group consisting of ethyl, CH₂Ph(*o*-OMe), CH₂Ph(*o*-OH), C(O)CH₂NH₂, C(O)CH(NH₂)(CH₂)₄NH₂, C(O)CH(NH₂)(CH₂)₃N(H)C(NH)(NH₂), 5-(1,2-dithiolan-3-yl)valeryl, and 2-cyano-1-phenylethyl;
R⁶ and R⁷ are each independently selected from the group consisting of H, methyl, and ethyl;
R⁸ is selected from the group consisting of H, methyl, ethyl, CH₂Ph(o-OMe), CH₂Ph(o-OH), C(O)CH₂NH₂, C(O)CH(NH₂)(CH₂)₄NH₂, C(O)CH(NH₂)(CH₂)₃N(H)C(NH)(NH₂), 5-(1,2-dithiolan-3-yl)valeryl, and 2-cyano-1-phenylethyl;
R⁹ is selected from the group consisting of CO₂R¹⁰, and COSR¹⁰;
R¹⁰ is selected from the group consisting of H, methyl, and ethyl; and
each R' is independently selected from the group consisting of H, and F;
or a stereoisomer or a pharmaceutically acceptable salt thereof.

2. The compound as claimed in any of the preceding claims, wherein
at least one of R⁶, R⁷, and R⁸ is each independently selected from the group consisting of methyl, and ethyl, and the other ones of R⁶, R⁷, and R⁸ are each independently selected from the group consisting of H, methyl, and ethyl;
R⁹ is CO₂R¹⁰; and
R¹⁰ is selected from the group consisting of methyl, and ethyl;
or a stereoisomer or a pharmaceutically acceptable salt thereof.

3. The compound as claimed in any of the preceding claims, wherein
R⁶ and R⁷ are each independently selected from the group consisting of methyl, and ethyl, or one of R⁶ and R⁷ is selected from the group consisting of methyl, and ethyl, and the other one of R⁶ and R⁷ is H;
R⁸ is selected from the group consisting of H, methyl, and ethyl;
R⁹ is CO₂R¹⁰; and
R¹⁰ is selected from the group consisting of methyl, and ethyl;
or a stereoisomer or a pharmaceutically acceptable salt thereof.

4. The compound as claimed in any of claims 1 - 2, wherein
R⁶ and R⁷ are both H;
R⁹ is CO₂R¹⁰; and
R¹⁰ is selected from the group consisting of methyl, and ethyl;
or a stereoisomer or a pharmaceutically acceptable salt thereof.

5. The compound as claimed in any of the preceding claims, wherein
R¹, R² and R⁵ are H;
one of R³ and R⁴ is selected from the group consisting of F, Cl, methyl, and ethyl, and the other one of R³ and R⁴ is selected from the group consisting of H, F, methyl, and ethyl;
R⁹ is CO₂R¹⁰; and
R¹⁰ is selected from the group consisting of methyl, and ethyl;
or a stereoisomer or a pharmaceutically acceptable salt thereof.

6. The compound as claimed in any of claims 1 - 4, wherein
R¹, R² and R⁵ are H; and
R³ and R⁴ are both halogen; or one of R³ and R⁴ is halogen or methyl, and the other one of R³ and R⁴ is H provided that R⁸ is selected from the group consisting of ethyl, CH₂Ph(*o*-OMe), CH₂Ph(*o*-OH), C(O)CH₂NH₂, C(O)CH(NH₂)(CH₂)₄NH₂, C(O)CH(NH₂)(CH₂)₃N(H)C(NH)(NH₂), 5-(1,2-dithiolan-3-yl)valeryl, and 2-cyano-1-phenylethyl, or provided that at least one of R⁶, and R⁷ is each independently selected from the group consisting of methyl, and ethyl, and the other one of R⁶, and R⁷ is selected from the group consisting of H, methyl, and ethyl;
or a stereoisomer or a pharmaceutically acceptable salt thereof.

7. The compound as claimed in any of claims 1 - 2 or claims 4 - 6, wherein
R¹, R² and R⁵ are H;
R³ and R⁴ are both F, or one of R³ and R⁴ is F and the other one of R³ and R⁴ is H;
R⁶ and R⁷ are each independently selected from the group consisting of H, and methyl;
R⁹ is CO₂R¹⁰; and
R¹⁰ is selected from the group consisting of methyl, and ethyl;
or a stereoisomer or a pharmaceutically acceptable salt thereof.

8. The compound as claimed in any of claims 1 - 2 or claims 4 - 7, wherein
R¹, R² and R⁵ are H;
R³ and R⁴ are both F;
R⁶ and R⁷ are each independently selected from the group consisting of H, and methyl;
R⁹ is CO₂R¹⁰; and
R¹⁰ is selected from the group consisting of methyl, and ethyl;
or a stereoisomer or a pharmaceutically acceptable salt thereof.

9. The compound as claimed in any of claims 1 - 6, wherein
R¹, R² and R⁵ are H;
one of R³ and R⁴ is methyl and the other one of R³ and R⁴ is H;
R⁹ is CO₂R¹⁰; and
R¹⁰ is selected from the group consisting of methyl, and ethyl;
or a stereoisomer or a pharmaceutically acceptable salt thereof.

10. The compound as claimed in any of claims 1 - 8, wherein
R¹, R² and R⁵ are H;
R³ and R⁴ are both F;
R⁸ is selected from the group consisting of methyl, and ethyl;
R⁹ is CO₂R¹⁰; and
R¹⁰ is selected from the group consisting of methyl, and ethyl;
or a stereoisomer or a pharmaceutically acceptable salt thereof or wherein the compound is selected from the group consisting of:
ethyl 3-(3,4-difluorophenyl)-1,2,4-trimethylazetidine-3-carboxylate (1);
ethyl 3-(3,4-difluorophenyl)-1-ethylazetidine-3-carboxylate (2);
ethyl 3-(3,4-difluorophenyl)-1-methylazetidine-3-carboxylate (3);
ethyl 3-(3,4-difluorophenyl)azetidine-3-carboxylate (4);
ethyl 3-(3-fluorophenyl)-2,4-dimethylazetidine-3-carboxylate (5);
ethyl 3-(3-fluorophenyl)-2-methylazetidine-3-carboxylate (6);
methyl 2-methyl-3-(3-methylphenyl)azetidine-3-carboxylate (8);
methyl 3-(3,4-difluorophenyl)-1-ethyl-2-methylazetidine-3-carboxylate (9);
methyl 3-(3,4-difluorophenyl)-2-methylazetidine-3-carboxylate (10);
ethyl 2-methyl-3-(3-methylphenyl)azetidine-3-carboxylate (11);
ethyl 1-ethyl-3-(4-methylphenyl)azetidine-3-carboxylate (12);
ethyl 3-(3,4-difluorophenyl)-2,4-dimethylazetidine-3-carboxylate (13);
methyl 3-(3,4-difluorophenyl)-1-ethyl-2,4-dimethylazetidine-3-carboxylate (14);
methyl 1-ethyl-2-methyl-3-(3-methylphenyl)azetidine-3-carboxylate (15);ethyl 3-(3-fluorophenyl)-1,2,4-trimethylazetidine-3-carboxylate (17) ;
ethyl 1-ethyl-2,4-dimethyl-3-(3-methylphenyl)azetidine-3-carboxylate (18);
ethyl 3-(3,4-difluorophenyl)-2-methylazetidine-3-carboxylate (19);
ethyl 1-ethyl-3-(3-methylphenyl)azetidine-3-carboxylate (21);
methyl 1-ethyl-3-(3-methoxyphenyl)-2,4-dimethylazetidine-3-carboxylate (22);
ethyl 1-ethyl-2,4-dimethyl-3-(4-methylphenyl)azetidine-3-carboxylate (23);
ethyl 2,4-dimethyl-3-(3-methylphenyl)azetidine-3-carboxylate (24);
methyl 3-(3,4-difluorophenyl)-2,4-dimethylazetidine-3-carboxylate (25);
ethyl 1,2-dimethyl-3-(3-methylphenyl)azetidine-3-carboxylate (26);
methyl 1-ethyl-2,4-dimethyl-3-(3-methylphenyl)azetidine-3-carboxylate (27);
methyl 1-ethyl-2-methyl-3-(4-methylphenyl)azetidine-3-carboxylate (28);
methyl 1-ethyl-3-(4-methoxyphenyl)-2,4-dimethylazetidine-3-carboxylate (29);
methyl 1-ethyl-3-(4-methoxyphenyl)-2-methylazetidine-3-carboxylate (30);
methyl 2,4-dimethyl-3-(4-methylphenyl)azetidine-3-carboxylate (31);
ethyl 3-(3,4-difluorophenyl)-1-ethyl-2-methylazetidine-3-carboxylate (32);
methyl 2-methyl-3-(4-methylphenyl)azetidine-3-carboxylate (33);
methyl 3-(4-methylphenyl)-1,2-dimethylazetidine-3-carboxylate (34);ethyl 1,2-dimethyl-3-(4-methylphenyl)azetidine-3-carboxylate (36); and
methyl 1-ethyl-3-(3-fluorophenyl)-2,4-dimethylazetidine-3-carboxylate (37);
or a stereoisomer or a pharmaceutically acceptable salt thereof.

11. A pharmaceutical composition comprising an effective amount of one or more compounds, or a stereoisomer or a pharmaceutically acceptable salt thereof, as defined in any of the preceding claims, together with one or more pharmaceutically acceptable excipient(s).

12. A compound, or a stereoisomer or pharmaceutically acceptable salt thereof, as defined in any of claims 1 - 10, for use as a medicament.

13. A compound, or a stereoisomer or pharmaceutically acceptable salt thereof, as defined in any of claims 1 - 10, for use in the treatment or prevention of drug addiction or a CNS related disease or condition.

14. A compound, or a stereoisomer or pharmaceutically acceptable salt thereof, as defined in any of claims 1 - 10, for the use in the treatment or prevention of drug addiction or a CNS related disease or condition as claimed in claim 13, wherein the drug addiction or the CNS related disease or condition is selected from the group consisting of stimulant addiction, ADHD, ADD, sluggish cognitive tempo, concentration deficit disorder, motivational or reward system dysfunction, autism spectrum disorder, disruptive, impulse control, and conduct disorders, anxiety disorders, eating disorders, depression, dysthymia, Alzheimer's disease, Parkinson's disease, hyperactivity, narcolepsy, and alcoholism.

15. A method for the preparation of a compound of formula (I), or pharmaceutically acceptable salt or a stereoisomer thereof, as defined in any one of claims 1 - 10, comprising:
(i) providing a compound of formula (II) 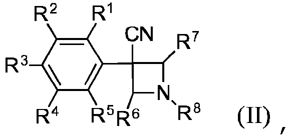 wherein
R¹, R², R³, R⁴, R⁵, R⁶, and R⁷ are as defined in claim 1; and R⁸ is methyl, ethyl, CH₂Ph(*o*-OMe), or a protecting group;
(ii) reacting the compound of formula (II) with one or more compounds each independently selected from the group consisting of acids, bases, and water; or
reacting the compound of formula (II) with methanol or ethanol, and a Lewis catalyst;
(iii) optionally performing an esterification with a compound of formula (III)
R¹¹-R" (III),
wherein
R¹¹ is methyl or ethyl; and
R'' is selected from the group consisting of halogen, SR‴, OR''', and a first activating group, wherein R‴ is H, or a second activating group, optionally in the presence of one or more activating group reactant(s) and/or one or more activating agent(s);
(iv) optionally performing one or more first deprotection reaction(s);
(v) optionally performing a transesterification with a compound of formula (III), wherein R¹¹ is methyl or ethyl; and R" is selected from the group consisting of SH, and OH, optionally in the presence of one or more activating group reactant(s) and/or one or more activating agent(s);
(vi) optionally performing an amidation with a compound of formula (IV)
R¹²-R^{8'} (IV),
wherein
R¹² is HO, R¹³O, or R¹³, wherein R¹³ is an activating group; and
R^{8'} is C(O)CH₂NHR¹⁴, C(O)CH(NHR¹⁴)(CH₂)₄NHR¹⁴, C (O)CH(NHR¹⁴)(CH₂)₃N(R¹⁴) C(NR¹⁴)(NHR¹⁴), or 5- (1,2-dithiolan-3-yl)valeryl, wherein each R¹⁴ is independently selected from the group consisting of H, and protecting groups, optionally in the presence of one or more activating group reactant(s) and/or one or more activating agent (s) ;
(vii) optionally performing a reductive alkylation with a compound of formula (V)
R^{8"}-CHO (V),
wherein
R^{8"} is H, or CH₃; and a reducing agent;
(viii) optionally performing an N-alkylation with a compound of formula (VI)
R^{8‴}-R¹⁵ (VI),
wherein
R^{8‴} is methyl, ethyl, (*o*-OMe)PhCH₂, or (Ph) CH (CH₂CN) ; and
R¹⁵ is selected from the group consisting of halogen, SR¹⁶, and OR¹⁶, wherein R¹⁶ is an activating group, optionally in the presence of one or more activating group reactant(s) and/or one or more activating agent(s);
(ix) optionally performing one or more second deprotection reaction(s);
to obtain the compound of formula (I), wherein R¹, R², R³, R⁴, R⁵, R⁶, R⁷, R⁸, R⁹ and R¹⁰ are as defined in claim 1; and
(x) optionally converting the compound of formula (I) to a pharmaceutically acceptable salt thereof.

## Patentansprüche

1. Verbindung von Formel (I) 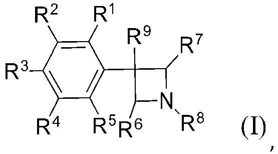 wobei
R¹, R² und R⁵ H sind;
R³ und R⁴ jeweils unabhängig aus der Gruppe bestehend aus H, Halogen, C₁₋₄-Alkyl, C₁₋₃-(Per)halogenalkyl, C₁₋₃-Alkoxy, C₁₋₃-(Per)halogenalkoxy und OH ausgewählt sind, mit der Maßgabe, dass, wenn einer von R³ oder R⁴ H ist, dann mindestens einer von R⁶und R⁷ unabhängig aus der Gruppe bestehend aus Methyl und Ethyl ausgewählt ist und der andere von R⁶ und R⁷ aus der Gruppe bestehend aus H, Methyl und Ethyl ausgewählt ist, oder sowohl R⁶ als auch R⁷ H sind, und R⁸ aus der Gruppe bestehend aus Ethyl, CH₂Ph(*o*-OMe), CH₂Ph(*o*-OH), C(O)CH₂NH₂, C(O)CH(NH₂)(CH₂)₄NH₂, C(O)CH(NH₂)(CH₂)₃N(H)C(NH)(NH₂), 5-(1,2-Dithiolan-3-yl)valeryl und 2-Cyano-1-phenylethyl ausgewählt ist;
R⁶ und R⁷ jeweils unabhängig aus der Gruppe bestehend aus H, Methyl und Ethyl ausgewählt sind;
R⁸ aus der Gruppe bestehend aus H, Methyl, Ethyl, CH₂Ph(*o*-OMe), CH₂Ph(*o*-OH), C(O)CH₂NH₂, C(O)CH(NH₂)(CH₂)₄NH₂, C(O)CH(NH₂)(CH₂)₃N(H)C(NH)(NH₂), 5-(1,2-Dithiolan-3-yl)valeryl und 2-Cyano-1-phenylethyl ausgewählt ist;
R⁹ aus der Gruppe bestehend aus CO₂R¹⁰ und COSR¹⁰ ausgewählt ist;
R¹⁰ aus der Gruppe bestehend aus H, Methyl und Ethyl ausgewählt ist; und
jeder R' unabhängig aus der Gruppe bestehend aus H und F ausgewählt ist;
oder ein Stereoisomer oder ein pharmazeutisch verträgliches Salz davon.

2. Verbindung nach einem der vorstehenden Ansprüche, wobei
mindestens einer von R⁶, R⁷ und R⁸ jeweils unabhängig aus der Gruppe bestehend aus Methyl und Ethyl ausgewählt ist und die anderen von R⁶, R⁷und R⁸ jeweils unabhängig aus der Gruppe bestehend aus H, Methyl und Ethyl ausgewählt sind;
R⁹ CO₂R¹⁰ ist; und
R¹⁰ aus der Gruppe bestehend aus Methyl und Ethyl ausgewählt ist;
oder ein Stereoisomer oder ein pharmazeutisch verträgliches Salz davon.

3. Verbindung nach einem der vorstehenden Ansprüche, wobei
R⁶ und R⁷ jeweils unabhängig aus der Gruppe bestehend aus Methyl und Ethyl ausgewählt sind oder entweder einer von R⁶ und R⁷ aus der Gruppe bestehend aus Methyl und Ethyl ausgewählt ist und der andere von R⁶ und R⁷ H ist;
R⁸ aus der Gruppe bestehend aus H, Methyl und Ethyl ausgewählt ist;
R⁹ CO₂R¹⁰ ist; und
R¹⁰ aus der Gruppe bestehend aus Methyl und Ethyl ausgewählt ist;
oder ein Stereoisomer oder ein pharmazeutisch verträgliches Salz davon.

4. Verbindung nach einem der Ansprüche 1-2, wobei
R⁶ und R⁷ beide H sind;
R⁹ CO₂R¹⁰ ist; und
R¹⁰ aus der Gruppe bestehend aus Methyl und Ethyl ausgewählt ist;
oder ein Stereoisomer oder ein pharmazeutisch verträgliches Salz davon.

5. Verbindung nach einem der vorstehenden Ansprüche, wobei
R¹, R² und R⁵ H sind;
einer von R³ und R⁴ aus der Gruppe bestehend aus F, Cl, Methyl und Ethyl ausgewählt ist und der andere von R³ und R⁴ aus der Gruppe bestehend aus H, F, Methyl und Ethyl ausgewählt ist;
R⁹ CO₂R¹⁰ ist; und
R¹⁰ aus der Gruppe bestehend aus Methyl und Ethyl ausgewählt ist;
oder ein Stereoisomer oder ein pharmazeutisch verträgliches Salz davon.

6. Verbindung nach einem der Ansprüche 1-4, wobei
R¹, R² und R⁵ H sind; und
R³ und R⁴ beide Halogen sind; oder einer von R³ und R⁴ Halogen oder Methyl ist und der andere von R³ und R⁴ H ist, vorausgesetzt, dass R⁸ aus der Gruppe bestehend aus Ethyl, CH₂Ph(*o-*OMe), CH₂Ph(*o*-OH), C(O)CH₂NH₂, C(O)CH(NH₂)(CH₂)₄NH₂, C(O)CH(NH₂)(CH₂)₃N(H)C(NH)(NH₂), 5-(1,2-Dithiolan-3-yl)valeryl und 2-Cyano-1-phenylethyl ausgewählt ist, oder vorausgesetzt, dass mindestens einer von R⁶ und R⁷ jeweils unabhängig aus der Gruppe bestehend aus Methyl und Ethyl ausgewählt ist und der andere von R⁶und R⁷ aus der Gruppe bestehend aus H, Methyl und Ethy ausgewählt istl;
oder ein Stereoisomer oder ein pharmazeutisch verträgliches Salz davon.

7. Verbindung nach einem der Ansprüche 1-2 oder Ansprüche 4-6, wobei
R¹, R² und R⁵ H sind;
R³ und R⁴ beide F sind oder entweder einer von R³ und R⁴ F ist und der andere von R³ und R⁴ H ist;
R⁶ und R⁷ jeweils unabhängig aus der Gruppe bestehend aus H und Methyl ausgewählt sind;
R⁹ CO₂R¹⁰ ist; und
R¹⁰ aus der Gruppe bestehend aus Methyl und Ethyl ausgewählt ist;
oder ein Stereoisomer oder ein pharmazeutisch verträgliches Salz davon.

8. Verbindung nach einem der Ansprüche 1-2 oder Ansprüche 4-7, wobei
R¹, R² und R⁵ H sind;
R³ und R⁴ beide F sind;
R⁶ und R⁷ jeweils unabhängig aus der Gruppe bestehend aus H und Methyl ausgewählt sind;
R⁹ CO₂R¹⁰ ist; und
R¹⁰ aus der Gruppe bestehend aus Methyl und Ethyl ausgewählt ist;
oder ein Stereoisomer oder ein pharmazeutisch verträgliches Salz davon.

9. Verbindung nach einem der Ansprüche 1-6, wobei
R¹, R² und R⁵ H sind;
einer von R³ und R⁴ Methyl ist und der andere von R³ und R⁴ H ist;
R⁹ CO₂R¹⁰ ist; und
R¹⁰ aus der Gruppe bestehend aus Methyl und Ethyl ausgewählt ist;
oder ein Stereoisomer oder ein pharmazeutisch verträgliches Salz davon.

10. Verbindung nach einem der Ansprüche 1-8, wobei
R¹, R² und R⁵ H sind;
R³ und R⁴ beide F sind;
R⁸ aus der Gruppe bestehend aus Methyl und Ethyl ausgewählt ist;
R⁹ CO₂R¹⁰ ist; und
R¹⁰ aus der Gruppe bestehend aus Methyl und Ethyl ausgewählt ist;
oder ein Stereoisomer oder pharmazeutisch verträgliches Salz davon oder wobei die Verbindung aus der Gruppe ausgewählt ist, bestehend aus:
Ethyl-3-(3,4-difluorphenyl)-1,2,4-trimethylazetidin-3-carboxylat (1);
Ethyl-3-(3,4-difluorphenyl)-1-ethylazetidin-3-carboxylat (2);
Ethyl-3-(3,4-difluorphenyl)-1-methylazetidin-3-carboxylat (3);
Ethyl-3-(3,4-difluorphenyl)azetidin-3-carboxylat (4);
Ethyl-3-(3-fluorphenyl)-2,4-dimethylazetidin-3-carboxylat (5);
Ethyl-3-(3-fluorphenyl)-2-methylazetidin-3-carboxylat (6);
Methyl-2-methyl-3-(3-methylphenyl)azetidin-3-carboxylat (8);
Methyl-3-(3,4-difluorphenyl)-1-ethyl-2-methylazetidin-3-carboxylat (9);
Methyl-3-(3,4-difluorphenyl)-2-methylazetidin-3-carboxylat (10);
Ethyl-2-methyl-3-(3-methylphenyl)azetidin-3-carboxylat (11);
Ethyl-1-ethyl-3-(4-methylphenyl)azetidin-3-carboxylat (12);
Ethyl-3-(3,4-difluorphenyl)-2,4-dimethylazetidin-3-carboxylat (13);
Methyl-3-(3,4-difluorphenyl)-1-ethyl-2,4-dimethylazetidin-3-carboxylat (14);
Methyl-1-ethyl-2-methyl-3-(3-methylphenyl)azetidin-3-carboxylat (15); Ethyl-3-(3-fluorphenyl)-1,2,4-trimethylazetidin-3-carboxylat (17);
Ethyl-1-ethyl-2,4-dimethyl-3-(3-methylphenyl)azetidin-3-carboxylat (18);
Ethyl-3-(3,4-difluorphenyl)-2-methylazetidin-3-carboxylat (19);
Ethyl-1-ethyl-3-(3-methylphenyl)azetidin-3-carboxylat (21);
Methyl-1-ethyl-3-(3-methoxyphenyl)-2,4-dimethylazetidin-3-carboxylat (22);
Ethyl-1-ethyl-2,4-dimethyl-3-(4-methylphenyl)azetidin-3-carboxylat (23);
Ethyl-2,4-dimethyl-3-(3-methylphenyl)azetidin-3-carboxylat (24);
Methyl-3-(3,4-difluorphenyl)-2,4-dimethylazetidin-3-carboxylat (25);
Ethyl-1,2-dimethyl-3-(3-methylphenyl)azetidin-3-carboxylat (26);
Methyl-1-ethyl-2,4-dimethyl-3-(3-methylphenyl)azetidin-3-carboxylat (27);
Methyl-1-ethyl-2-methyl-3-(4-methylphenyl)azetidin-3-carboxylat (28);
Methyl-1-ethyl-3-(4-methoxyphenyl)-2,4-dimethylazetidin-3-carboxylat (29);
Methyl-1-ethyl-3-(4-methoxyphenyl)-2-methylazetidin-3-carboxylat (30);
Methyl-2,4-dimethyl-3-(4-methylphenyl)azetidin-3-carboxylat (31);
Ethyl-3-(3,4-difluorphenyl)-1-ethyl-2-methylazetidin-3-carboxylat (32);
Methyl-2-methyl-3-(4-methylphenyl)azetidin-3-carboxylat (33);
Methyl-3-(4-methylphenyl)-1,2-dimethylazetidin-3-carboxylat (34); Ethyl-1,2-dimethyl-3-(4-methylphenyl)azetidin-3-carboxylat (36); und
Methyl-1-ethyl-3-(3-fluorphenyl)-2,4-dimethylazetidin-3-carboxylat (37);
oder ein Stereoisomer oder ein pharmazeutisch verträgliches Salz davon.

11. Pharmazeutische Zusammensetzung, umfassend eine wirksame Menge von einer oder mehreren Verbindungen oder eines Stereoisomers oder eines pharmazeutisch verträglichen Salzes davon, wie in einem der vorstehenden Ansprüche definiert, zusammen mit einem oder mehreren pharmazeutisch verträglichen Hilfsstoff(en).

12. Verbindung oder ein Stereoisomer oder pharmazeutisch verträgliches Salz davon, wie in einem von Ansprüchen 1-10 definiert, zur Verwendung als ein Medikament.

13. Verbindung oder ein Stereoisomer oder pharmazeutisch verträgliches Salz davon, wie in einem der Ansprüche 1-10 definiert, zur Verwendung bei der Behandlung oder Prävention von Drogenabhängigkeit oder einer Erkrankung oder eines Zustands in Zusammenhang mit dem ZNS.

14. Verbindung oder ein Stereoisomer oder pharmazeutisch verträgliches Salz davon, wie in einem der Ansprüche 1-10 definiert, zur Verwendung bei der Behandlung oder Prävention von Drogenabhängigkeit oder einer Erkrankung oder einem Zustand in Zusammenhang mit dem ZNS nach Anspruch 13, wobei die Drogenabhängigkeit oder die Erkrankung oder der Zustand in Zusammenhang mit dem ZNS aus der Gruppe bestehend aus Stimulanzienabhängigkeit, ADHS, ADS, trägem kognitiven Tempo, Konzentrationsstörung, Dysfunktion des Motivations- oder Belohnungssystems, Autismus-Spektrum-Störung, disruptiven Störungen, Impulskontrollstörungen und Verhaltensstörungen, Angststörungen, Essstörungen, Depression, Dysthymie, Alzheimer-Krankheit, Parkinson-Krankheit, Hyperaktivität, Narkolepsie und Alkoholismus ausgewählt ist.

15. Verfahren zur Herstellung einer Verbindung der Formel (I) oder pharmazeutisch akzeptablen Salzes oder eines Stereoisomers davon, wie in einem der Ansprüche 1-10 definiert, umfassend:
(i) Bereitstellen einer Verbindung der Formel (II), 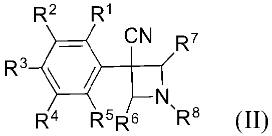 wobei
R¹, R², R³, R⁴, R⁵, R⁶und R⁷ wie in Anspruch 1 definiert sind; und R⁸ Methyl, Ethyl, CH₂Ph(*o*-OMe) oder eine Schutzgruppe ist;
(ii) Umsetzen der Verbindung der Formel (II) mit einer oder mehreren Verbindungen, die jeweils unabhängig aus der Gruppe bestehend aus Säuren, Basen und Wasser ausgewählt sind; oder
Umsetzen der Verbindung der Formel (II) mit Methanol oder Ethanol und einem Lewis-Katalysator;
(iii) optional Durchführen einer Veresterung mit einer Verbindung der Formel (III)
R¹¹-R" (III),
wobei
R¹¹ Methyl oder Ethyl ist; und
R"t aus der Gruppe bestehend aus Halogen, SR‴, OR‴ und einer ersten aktivierenden Gruppe ausgewählt is, wobei R‴ H ist, oder einer zweiten aktivierenden Gruppe, optional in Gegenwart eines oder mehrerer Reaktanten mit aktivierender Gruppe und/oder eines oder mehrerer aktivierenden Mittel;
(iv) optional Durchführen einer oder mehrerer erster Entschützungsreaktionen;
(v) optional Durchführen einer Umesterung mit einer Verbindung der Formel (III), wobei R¹¹ Methyl oder Ethyl ist; und R" aus der Gruppe bestehend aus SH und OH ausgewählt ist, optional in Gegenwart eines oder mehrerer Reaktanten mit aktivierender Gruppe und/oder eines oder mehrerer aktivierenden Mittel;
(vi) optional Durchführen einer Amidierung mit einer Verbindung der Formel (IV)
R¹²-R^{8'} (IV),
wobei
R¹² HO, R¹³O oder R¹³ ist, wobei R¹³ eine aktivierende Gruppe ist; und
R^{8'} C(O)CH₂NHR¹⁴, C(O)CH(NHR¹⁴)(CH₂)₄NHR¹⁴, C(O)CH(NHR¹⁴)(CH₂)₃N(R¹⁴)C(NR¹⁴)(NHR¹⁴) oder 5-(1,2-Dithiolan-3-yl)valeryl ist, wobei jeder R¹⁴ unabhängig aus der Gruppe bestehend aus H und Schutzgruppen ausgewählt ist, optional in Gegenwart eines oder mehrerer Reaktanten mit aktivierender Gruppe und/oder eines oder mehrerer aktivierenden Mitte;
(vii) optional Durchführen einer reduktiven Alkylierung mit einer Verbindung der Formel (V)
R^{8''}-CHO (V),
wobei
R^{8''} H oder CH₃ ist; und einem Reduktionsmittel;
(viii) optional Durchführen einer *N*-Alkylierung mit einer Verbindung der Formel (VI)
R^{8''***'***}-R¹⁵ (VI),
wobei
R^{8‴} Methyl, Ethyl, (*o*-OMe)PhCH₂ oder (Ph)CH(CH₂CN) ist; und
R¹⁵ aus der Gruppe bestehend aus Halogen, SR¹⁶ und OR¹⁶ ausgewählt ist, wobei R¹⁶ eine aktivierende Gruppe ist, optional in Gegenwart eines oder mehrerer Reaktanten mit aktivierender Gruppe und/oder eines oder mehrerer aktivierenden Mittel;
(ix) optional Durchführen einer zweiten oder mehrerer zweiter Entschützungsreaktionen;
um die Verbindung der Formel (I) zu erhalten, wobei R¹, R², R³, R⁴, R⁵, R⁶, R⁷, R⁸, R⁹ und R¹⁰ wie in Anspruch 1 definiert sind; und
(x) optional Umwandeln der Verbindung der Formel (I) in ein pharmazeutisch verträgliches Salz davon.

## Revendications

1. Composé de formule (I) 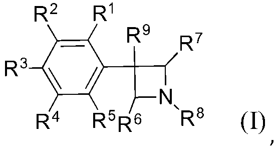 dans lequel
R¹, R² et R⁵ sont H ;
R³ et R⁴ sont chacun indépendamment choisis dans le groupe consistant en H, halogène, alkyle en C₁₋₄, (per)haloalkyle en C₁₋₃, alcoxy en C₁₋₃, (per)haloalcoxy en C₁₋₃et OH, à condition que l'un de R³ ou R⁴ soit H alors au moins l'un de R⁶ et R⁷ est chacun choisi indépendamment dans le groupe consistant en méthyle et éthyle, et l'autre de R⁶, et R⁷ est choisi dans le groupe consistant en H, méthyle et éthyle, ou R⁶ et R⁷ sont tous les deux H, et R⁸ est choisi dans le groupe consistant en éthyle, CH₂Ph(*o*-OMe), CH₂Ph(*o*-OH), C(O)CH₂NH₂, C(O)CH(NH₂)(CH₂)₄NH₂, C(O)CH(NH₂)(CH₂)₃ N(H)C(NH)(NH₂), 5-(1,2-dithiolan-3-yl)valéryle et 2-cyano-1-phényléthyle ;
R⁶ et R⁷ sont chacun choisis indépendamment dans le groupe consistant en H, méthyle et éthyle ;
R⁸ est choisi dans le groupe consistant en H, méthyle, éthyle, CH₂Ph(*o*-OMe), CH₂Ph(*o*-OH), C(O)CH₂NH₂, C(O)CH(NH₂)(CH₂)₄NH₂, C(O)CH(NH₂)(CH₂)₃ N(H)C(NH)(NH₂), 5-(1,2-dithiolan-3-yl)valéryle et 2-cyano-1-phényléthyle ;
R⁹ est choisi dans le groupe consistant en CO₂R¹⁰ et COSR¹⁰ ;
R¹⁰ est choisi dans le groupe consistant en H, méthyle et éthyle; et
chaque R' est choisi indépendamment dans le groupe consistant en H et F ;
ou un stéréoisomère ou un sel pharmaceutiquement acceptable de celui-ci.

2. Composé selon l'une quelconque des revendications précédentes, dans lequel
au moins l'un de R⁶, R⁷et R⁸ est choisi indépendamment dans le groupe consistant en méthyle et éthyle, et les autres de R⁶, R⁷et R⁸ sont choisis indépendamment dans le groupe consistant en H, méthyle et éthyle ;
R⁹ est CO₂R¹⁰; et
R¹⁰ est choisi dans le groupe consistant en méthyle et éthyle ;
ou un stéréoisomère ou un sel pharmaceutiquement acceptable de celui-ci.

3. Composé selon l'une quelconque des revendications précédentes, dans lequel
R⁶ et R⁷ sont chacun choisis indépendamment dans le groupe consistant en méthyle et éthyle, ou l'un de R⁶ et R⁷ est choisi dans le groupe consistant en méthyle et éthyle, et l'autre de R⁶ et R⁷ est H ;
R⁸ est choisi dans le groupe consistant en H, méthyle et éthyle ;
R⁹ est CO₂R¹⁰ ; et
R¹⁰ est choisi dans le groupe consistant en méthyle et éthyle ;
ou un stéréoisomère ou un sel pharmaceutiquement acceptable de celui-ci.

4. Composé selon l'une quelconque des revendications 1 - 2, dans lequel
R⁶ et R⁷ sont tous les deux H ;
R⁹ est CO₂R¹⁰ ; et
R¹⁰ est choisi dans le groupe consistant en méthyle et éthyle ;
ou un stéréoisomère ou un sel pharmaceutiquement acceptable de celui-ci.

5. Composé selon l'une quelconque des revendications précédentes, dans lequel
R¹, R² et R⁵ sont H ;
l'un de R³ et R⁴ est choisi dans le groupe consistant en F, Cl, méthyle et éthyle, et l'autre de R³ et R⁴ est choisi dans le groupe consistant en H, F, méthyle et éthyle ;
R⁹ est CO₂R¹⁰ ; et
R¹⁰ est choisi dans le groupe consistant en méthyle et éthyle ;
ou un stéréoisomère ou un sel pharmaceutiquement acceptable de celui-ci.

6. Composé selon l'une quelconque des revendications 1 - 4, dans lequel
R¹, R² et R⁵ sont H ; et
R³ et R⁴ sont tous les deux halogène ; ou l'un de R³ et R⁴ est halogène ou méthyle, et l'autre de R³ et R⁴ est H à condition que R⁸ soit choisi dans le groupe consistant en éthyle, CH₂Ph(*o*-OMe), CH₂Ph(*o*-OH), C(O)CH₂NH₂, C(O)CH(NH₂)(CH₂)₄NH₂, C(O)CH(NH₂)(CH₂)₃ N(H)C(NH)(NH₂), 5-(1,2-dithiolan-3-yl)valéryle et 2-cyano-1-phényléthyle, ou à condition qu'au moins l'un de R⁶et R⁷ soit choisi indépendamment dans le groupe consistant en méthyle et éthyle, et l'autre de R⁶et R⁷ soit choisi dans le groupe consistant en H, méthyle et éthyle ;
ou un stéréoisomère ou un sel pharmaceutiquement acceptable de celui-ci.

7. Composé selon l'une quelconque des revendications 1 - 2 ou des revendications 4 - 6, dans lequel
R¹, R² et R⁵ sont H ;
R³ et R⁴ sont tous les deux F, ou l'un de R³ et R⁴ est F et l'autre de R³ et R⁴ est H ;
R⁶ et R⁷ sont chacun choisis indépendamment dans le groupe consistant en H et méthyle ;
R⁹ est CO₂R¹⁰ ; et
R¹⁰ est choisi dans le groupe consistant en méthyle et éthyle ;
ou un stéréoisomère ou un sel pharmaceutiquement acceptable de celui-ci.

8. Composé selon l'une quelconque des revendications 1 - 2 ou des revendications 4 - 7, dans lequel
R¹, R² et R⁵ sont H ;
R³et R⁴ sont tous les deux F ;
R⁶ et R⁷ sont chacun choisis indépendamment dans le groupe consistant en H et méthyle ;
R⁹ est CO₂R¹⁰ ; et
R¹⁰ est choisi dans le groupe consistant en méthyle et éthyle ;
ou un stéréoisomère ou un sel pharmaceutiquement acceptable de celui-ci.

9. Composé selon l'une quelconque des revendications 1 - 6, dans lequel
R¹, R² et R⁵ sont H ;
l'un de R³ et R⁴ est méthyle et l'autre de R³ et R⁴ est H ;
R⁹ est CO₂R¹⁰ ; et
R¹⁰ est choisi dans le groupe consistant en méthyle et éthyle ;
ou un stéréoisomère ou un sel pharmaceutiquement acceptable de celui-ci.

10. Composé selon l'une quelconque des revendications 1 - 8, dans lequel
R¹, R² et R⁵ sont H ;
R³ et R⁴ sont tous les deux F ;
R⁸ est choisi dans le groupe consistant en méthyle et éthyle ;
R⁹ est CO₂R¹⁰ ; et
R¹⁰ est choisi dans le groupe consistant en méthyle et éthyle ;
ou un stéréoisomère ou un sel pharmaceutiquement acceptable de celui-ci ou dans lequel le composé est choisi dans le groupe consistant en :
3-(3,4-difluorophényl)-1,2,4-triméthylazétidine-3-carboxylate d'éthyle (1) ;
3-(3,4-difluorophényl)-1-éthylazétidine-3-carboxylate d'éthyle (2) ;
3-(3,4-difluorophényl)-1-méthylazétidine-3-carboxylate d'éthyle (3) ;
3-(3,4-difluorophényl)azétidine-3-carboxylate d'éthyle (4) ;
3-(3-fluorophényl)-2,4-diméthylazétidine-3-carboxylate d'éthyle (5) ;
3-(3-fluorophényl)-2-méthylazétidine-3-carboxylate d'éthyle (6) ;
2-méthyl-3-(3-méthylphényl)azétidine-3-carboxylate de méthyle (8) ;
3-(3,4-difluorophényl)-1-éthyl-2-méthylazétidine-3-carboxylate de méthyle (9) ;
3-(3,4-difluorophényl)-2-méthylazétidine-3-carboxylate de méthyle (10) ;
2-méthyl-3-(3-méthylphényl)azétidine-3-carboxylate d'éthyle (11) ;
1-éthyl-3-(4-méthylphényl)azétidine-3-carboxylate d'éthyle (12) ;
3-(3,4-difluorophényl)-2,4-diméthylazétidine-3-carboxylate d'éthyle (13) ;
3-(3,4-difluorophényl)-1-éthyl-2,4-diméthylazétidine-3-carboxylate de méthyle (14) ;
1-éthyl-2-méthyl-3-(3-méthylphényl)azétidine-3-carboxylate de méthyle (15) ; 3-(3-fluorophényl)-1,2,4-triméthylazétidine-3-carboxylate d'éthyle (17) ;
1-éthyl-2,4-diméthyl-3-(3-méthylphényl)azétidine-3-carboxylate d'éthyle (18) ;
3-(3,4-difluorophényl)-2-méthylazétidine-3-carboxylate d'éthyle (19) ;
1-éthyl-3-(3-méthylphényl)azétidine-3-carboxylate d'éthyle (21) ;
1-éthyl-3-(3-méthoxyphényl)-2,4-diméthylazétidine-3-carboxylate de méthyle (22) ;
1-éthyl-2,4-diméthyl-3-(4-méthylphényl)azétidine-3-carboxylate d'éthyle (23) ;
2,4-diméthyl-3-(3-méthylphényl)azétidine-3-carboxylate d'éthyle (24) ;
3-(3,4-difluorophényl)-2,4-diméthylazétidine-3-carboxylate de méthyle (25) ;
1,2-diméthyl-3-(3-méthylphényl)azétidine-3-carboxylate d'éthyle (26) ;
1-éthyl-2,4-diméthyl-3-(3-méthylphényl)azétidine-3-carboxylate de méthyle (27) ;
1-éthyl-2-méthyl-3-(4-méthylphényl)azétidine-3-carboxylate de méthyle (28) ;
1-éthyl-3-(4-méthoxyphényl)-2,4-diméthylazétidine-3-carboxylate de méthyle (29) ;
1-éthyl-3-(4-méthoxyphényl)-2-méthylazétidine-3-carboxylate de méthyle (30) ;
2,4-diméthyl-3-(4-méthylphényl)azétidine-3-carboxylate de méthyle (31) ;
3-(3,4-difluorophényl)-1-éthyl-2-méthylazétidine-3-carboxylate d'éthyle (32) ;
2-méthyl-3-(4-méthylphényl)azétidine-3-carboxylate (33) de méthyle ;
3-(4-méthylphényl)-1,2-diméthylazétidine-3-carboxylate de méthyle (34) ; 1,2-diméthyl-3-(4-méthylphényl)azétidine-3-carboxylate d'éthyle (36) ; et
1-éthyl-3-(3-fluorophényl)-2,4-diméthylazétidine-3-carboxylate de méthyle (37) ;
ou un stéréoisomère ou un sel pharmaceutiquement acceptable de celui-ci.

11. Composition pharmaceutique comprenant une quantité efficace d'un ou de plusieurs composés, ou d'un stéréoisomère ou d'un sel pharmaceutiquement acceptable de celui-ci, tel que défini dans l'une quelconque des revendications précédentes, en conjugaison avec un ou plusieurs excipients pharmaceutiquement acceptables.

12. Composé ou stéréoisomère ou sel pharmaceutiquement acceptable de celui-ci, tel que défini dans l'une quelconque des revendications 1 - 10, destiné à être utilisé comme médicament.

13. Composé ou stéréoisomère ou sel pharmaceutiquement acceptable de celui-ci, tel que défini dans l'une quelconque des revendications 1 - 10, destiné à être utilisé dans le traitement ou la prévention de la toxicomanie ou d'une maladie ou affection liée au SNC.

14. Composé ou stéréoisomère ou sel pharmaceutiquement acceptable de celui-ci, tel que défini dans l'une quelconque des revendications 1 - 10, destiné à être utilisé dans le traitement ou la prévention de la toxicomanie ou d'une maladie ou affection liée au SNC tel que revendiqué dans la revendication 13, dans lequel la toxicomanie ou la maladie ou affection liée au SNC est choisie dans le groupe consistant en la dépendance aux stimulants, le TDAH, le TDA, le tempo cognitif lent, le trouble déficitaire de la concentration, le dysfonctionnement du système motivationnel ou de récompense, le trouble du spectre autistique, les troubles disruptifs, le contrôle des impulsions et du comportement, les troubles anxieux, les troubles de l'alimentation, la dépression, la dysthymie, la maladie d'Alzheimer, la maladie de Parkinson, l'hyperactivité, la narcolepsie et l'alcoolisme.

15. Procédé de préparation d'un composé de formule (I), ou d'un sel pharmaceutiquement acceptable ou d'un stéréoisomère de celui-ci, tel que défini dans l'une quelconque des revendications 1 - 10, comprenant :
(i) la fourniture d'un composé de formule (II) 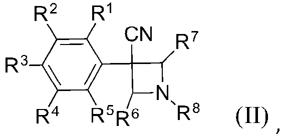 dans lequel
R¹, R², R³, R⁴, R⁵, R⁶ et R⁷ sont tels que définis dans la revendication 1 ; et R⁸ est méthyle, éthyle, CH₂Ph(*o*-OMe) ou un groupe protecteur ;
(ii) la réaction du composé de formule (II) avec un ou plusieurs composés, chacun étant choisi indépendamment dans le groupe consistant en acides, bases et eau ; ou
la mise en réaction du composé de formule (II) avec du méthanol ou de l'éthanol et un catalyseur de Lewis ;
(iii) éventuellement la réalisation d'une estérification avec un composé de formule (III)
R¹¹-R" (III),
dans lequel
R¹¹ est méthyle ou éthyle, et
R'' est choisi dans le groupe consistant en halogène, SR‴, OR‴ et un premier groupe activateur, dans lequel R‴ est H ou un deuxième groupe activateur, éventuellement en la présence d'un ou de plusieurs réactifs de groupe activateur et/ou un ou plusieurs agents activateurs ;
(iv) éventuellement la réalisation d'une ou de plusieurs premières réactions de déprotection ;
(v) éventuellement la réalisation d'une transestérification avec un composé de formule (III), dans lequel R¹¹ est méthyle ou éthyle ; et R" est choisi dans le groupe consistant en SH et OH, éventuellement en la présence d'un ou de plusieurs réactifs de groupe activateur et/ou d'un ou de plusieurs agents activateurs ;
(vi) éventuellement la réalisation d'une amidation avec un composé de formule (IV)
R¹²-R^{8'} (IV),
dans lequel
R¹² est HO, R¹³O ou R¹³, dans lequel R¹³ est un groupe activateur ; et
R^{8'} est C(O)CH₂NHR¹⁴, C(O)CH(NHR¹⁴)(CH₂)₄NHR¹⁴, C(O)CH(NHR¹⁴)(CH₂)₃N(R¹⁴)C(NR¹⁴)(NHR¹⁴) ou 5-(1,2-dithiolan-3-yl)valéryle, dans lequel chaque R¹⁴ est choisi indépendamment dans le groupe consistant en H et des groupes protecteurs, éventuellement en la présence d'un ou de plusieurs réactifs de groupe activateur et/ou d'un ou de plusieurs agents activateurs ;
(vii) éventuellement la réalisation d'une alkylation réductrice avec un composé de formule (V)
R^{8''}-CHO (V),
dans lequel
R^{8''} est H ou CH₃ ; et un agent réducteur ;
(viii) éventuellement la réalisation d'une *N*-alkylation avec un composé de formule (VI)
R^{8‴}-R¹⁵ (VI),
dans lequel
R^{8'''} est méthyle, éthyle, (*o*-OMe)PhCH₂ ou (Ph)CH(CH₂CN) ; et
R¹⁵ est choisi dans le groupe consistant en halogène, SR¹⁶et OR¹⁶, dans lequel R¹⁶ est un groupe activateur, éventuellement en la présence d'un ou de plusieurs réactifs de groupe activateur et/ou d'un ou de plusieurs agents activateurs ;
(ix) éventuellement la réalisation d'une ou de plusieurs secondes réactions de déprotection ;
pour obtenir le composé de formule (I), dans lequel R¹, R², R³, R⁴, R⁵, R⁶, R⁷, R⁸, R⁹ et R¹⁰ sont tels que définis dans la revendication 1 ; et
(x) éventuellement la conversion du composé de formule (I) en un sel pharmaceutiquement acceptable de celui-ci.
